(19) Europäisches Patentamt / European Patent Office / Office européen des brevets

(11) **EP 3 655 407 B1**

(12) # FASCICULE DE BREVET EUROPEEN

(45) Date de publication et mention de la délivrance du brevet:
**04.08.2021 Bulletin 2021/31**

(21) Numéro de dépôt: **18745561.3**

(22) Date de dépôt: **20.07.2018**

(51) Int Cl.:
***C07D 475/04*** (2006.01) ***C07D 487/22*** (2006.01)
***A61P 35/04*** (2006.01) ***A61K 31/409*** (2006.01)
***G01N 21/64*** (2006.01)

(86) Numéro de dépôt international:
**PCT/EP2018/069836**

(87) Numéro de publication internationale:
**WO 2019/016397 (24.01.2019 Gazette 2019/04)**

(54) **CONJUGUÉ DE PYROPHÉOPHORBIDE ET SON UTILISATION DANS LE TRAITEMENT DU CANCER ET COMME MARQUEUR FLUORESCENT**

CONJUGUATE OF PYROPHEOPHORBIDE AND ITS USE IN THE TREATMENT OF CANCER AND AS FLUORESCENT LABEL

PYROPHEOPHORBID-KONJUGAT UND DESSEN VERWENDUNG ZUR BEHANDLUNG VON KREBS UND ALS FLUORESZIERENDEN MARKER.

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.07.2017 FR 1756924**

(43) Date de publication de la demande:
**27.05.2020 Bulletin 2020/22**

(73) Titulaires:
- **INSERM (Institut National de la Santé et de la Recherche Médicale) 75013 Paris (FR)**
- **Centre Hospitalier Régional et Universitaire de Lille 59000 Lille (FR)**
- **Université de Lille 59000 Lille (FR)**
- **Centre National de la Recherche Scientifique 75016 Paris (FR)**
- **Université de Lorraine 54052 Nancy Cedex (FR)**
- **Institut Pasteur De Lille 59000 Lille (FR)**

(72) Inventeurs:
- **AZAIS, Henri 59000 Lille (FR)**
- **COLLINET, Pierre 59130 Lambersart (FR)**
- **DELHEM-FELLAHI, Nadira 59700 Marcq-En-Baroeul (FR)**
- **MORALES, Olivier 59800 Lille (FR)**
- **MORDON, Serge 59420 Mouvaux (FR)**
- **FROCHOT, Céline 54220 Malzeville (FR)**
- **VANDERESSE, Régis 54840 Sexey Les Bois (FR)**
- **STALLIVIERI, Aurélie 14000 Caen (FR)**

(74) Mandataire: **Plasseraud IP 66, rue de la Chaussée d'Antin 75440 Paris Cedex 09 (FR)**

(56) Documents cités:
**EP-A1- 2 431 366 WO-A2-2004/005289**

- **YOU HYUN ET AL: "Pheophorbide-aconjugates with cancer-targeting moieties for targeted photodynamic cancer therapy", BIOORGANIC & MEDICINAL CHEMISTRY, PERGAMON, GB, vol. 23, no. 7, 16 février 2015 (2015-02-16), pages 1453-1462, XP029204983, ISSN: 0968-0896, DOI: 10.1016/J.BMC.2015.02.014 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- SCHNEIDER R ET AL: "Design, synthesis, and biological evaluation of folic acid targeted tetraphenylporphyrin as novel photosensitizers for selective photodynamic therapy", BIOORGANIC & MEDICINAL CHEMISTRY, PERGAMON, GB, vol. 13, no. 8, 15 avril 2005 (2005-04-15) , pages 2799-2808, XP027637588, ISSN: 0968-0896 [extrait le 2005-04-15] cité dans la demande

SEQUENCE LISTING

<110> Prodynov

<120> Conjugués de pyropheophorbide et leurs utilisations

<130> B2549PCOO

<160> 10

<170> PatentIn version 3.5

<210> 1
<211> 20
<212> DNA
<213> Artificial Sequence

<220>
<223> FOLR1 forward

<400> 1
aggtgccatc tctccacagt 20

<210> 2
<211> 20
<212> DNA
<213> Artificial Sequence

<220>
<223> FOLR1 reverse

<400> 2
gaggacaagt tgcatgagca 20

<210> 3
<211> 20
<212> DNA
<213> Artificial Sequence

<220>
<223> FOLR2 forward

<400> 3
ctggctcctt ggctgagttc 20

<210> 4
<211> 18

<212> DNA
<213> Artificial Sequence

<220>
<223> FOLR2 reverse

<400> 4
gcccagcctg gttatcca 18

<210> 5
<211> 20
<212> DNA
<213> Artificial Sequence

<220>
<223> 18S forward

<400> 5
tcaagaacga aagtcggagg 20

<210> 6
<211> 20
<212> DNA
<213> Artificial Sequence

<220>
<223> 18S reverse

<400> 6
ggacatctaa gggcatcaca 20

<210> 7
<211> 26
<212> DNA
<213> Artificial Sequence

<220>
<223> GAPDH forward

<400> 7
gccaaggtca tccatgacaa ctttgg 26

<210> 8
<211> 26
<212> DNA
<213> Artificial Sequence

<220>
<223> GAPDH reverse

<400> 8
gcctgcttca ccaccttctt gatgtc 26

<210> 9
<211> 18
<212> DNA
<213> Artificial Sequence

<220>
<223> HPRT forward

<400> 9
ccctggcgtc gtgattag 18

<210> 10
<211> 18
<212> DNA
<213> Artificial Sequence

<220>
<223> HPRT reverse

<400> 10
atggcctccc atctcctt 18

## Description

La présente invention concerne le domaine des photosensibilisateurs et, plus particulièrement, leurs utilisations dans des protocoles de thérapie photodynamique pour traiter un cancer, notamment un cancer des ovaires.

ARRIERE-PLAN TECHOLOGIQUE DE L'INVENTION

Le cancer de l'ovaire représente 4500 nouveaux cas chaque année en France. Le mauvais pronostic de la maladie est lié au retard diagnostique puisque la majorité des cas sont diagnostiqués aux stades III et IV de la Fédération Internationale de Gynécologie Obstétrique (FIGO), et le taux de survie diminue avec l'avancement de la maladie. Le cancer ovarien est responsable de la majorité des décès par cancer gynécologique aux États-Unis et en Europe occidentale. Environ 3500 décès lui sont attribués chaque année en France.

La maladie évolue fréquemment vers l'apparition de métastases sous la forme d'une carcinose péritonéale, ce qui correspond à la présence de nombreuses tumeurs à la surface des organes de la cavité abdomino-pelvienne. Le péritoine est une membrane séreuse continue (formée par une couche simple de cellules mésothéliales) qui tapisse l'abdomen, le pelvis et les viscères, délimitant l'espace virtuel de la cavité péritonéale. On distingue le péritoine viscéral (qui tapisse l'extérieur des organes) et le péritoine pariétal (qui tapisse la face interne des parois de l'abdomen).

La prise en charge actuelle associe, lorsqu'elle est possible, une chirurgie à une chimiothérapie reposant sur l'utilisation de sels de platine, et dans certains cas à des thérapies ciblées. Il est admis que l'absence de lésions résiduelles après la chirurgie est le facteur principal de bon pronostic. La capacité du traitement chirurgical à éradiquer l'ensemble des implants tumoraux est donc décisive. La chimiothérapie systémique adjuvante ou néo-adjuvante a permis une amélioration des taux de survie à cinq ans, surtout dans les stades précoces : 81% pour les stades I et II après trois cycles de chimiothérapie associant carboplatine et paclitaxel. Dans le cadre d'une cytoréduction maximale et en complément d'une chirurgie de réduction tumorale macroscopiquement complète, des stratégies thérapeutiques sont envisageables telles que la chimiothérapie intrapéritonéale et la thérapie photodynamique intrapéritonéale. Malgré de nombreux essais cliniques, le bénéfice de la chimiothérapie intrapéritonéale n'est pas démontré dans cette indication et cette option n'est pas recommandée en dehors d'essai clinique.

Contrairement à la chimiothérapie intrapéritonéale, avec ou sans hyperthermie, le recours à un photosensibilisateur ciblant de manière sélective les lésions précoces, et uniquement ces lésions, permettrait de réduire la toxicité du traitement, l'action de la lumière n'ayant lieu qu'en présence de photosensibilisateurs au sein du tissu tumoral. Il pourrait également permettre de traiter les métastases microscopiques qui sont ignorées lors de la chirurgie. Ainsi, le recours à des photosensibilisateurs plus spécifiques ciblant des récepteurs surexprimés par les cellules tumorales ovariennes pourrait renforcer l'efficacité de la thérapie photodynamique (PDT).

Dans ce contexte, Schneider et al. (Bioorganic & Médicinal Chemistry, 2005, 13, 2799-2808) ont synthétisé un photosensibilisateur comprenant un motif triphénylporphyrinyl conjugué avec l'acide folique (TPP-FA) présentant à la fois des propriétés fluorescentes et antiprolifératives intéressantes pour une application en thérapie photodynamique. Plus particulièrement, Azaïs et al. (Photodiagnosis and Photodynamic Therapy, 2016, 13, 130-138) ont démontré sur modèle animal que ce photosensibilisateur était notamment spécifique des métastases péritonéales des cancers épithéliaux de l'ovaire et ont ainsi suggéré son utilisation dans le développement de protocoles de thérapie photodynamique intrapéritonéale non toxiques pour le patient. Toutefois, ce photosensibilisateur présente d'une part une fluorescence très faible empêchant ainsi sa détection avec les dispositifs médicaux couramment utilisés par les gynécologues et d'autre part une faible stabilité.

You et al. (Bioorganic & Médicinal Chemistry, 2015, 23, 1453-1462) ont également développé des conjugués de phéophorbide-a, notamment un photosensibilisateur comprenant un motif phéophorbide-a conjugué avec l'acide folique *via* un bras espaceur de type polyéthylène glycol (Pheo-PEG-FA). You et al. ont notamment montré que ce photosensibilisateur (Pheo-PEG-FA) ciblait les récepteurs aux folates, et pouvait être ainsi utilisé dans des protocoles de thérapie photodynamique pour traiter les cancers surexprimant les récepteurs à l'acide folique au vu de ses propriétés fluorescentes. Toutefois ce photosensibilisateur présente une fluorescence relativement modérée.

Ainsi, il existe aujourd'hui un réel besoin de développer de nouveaux photosensibilisateurs pour être utilisés en thérapie photodynamique. Ces photosensibilisateurs doivent à la fois présenter une bonne efficacité thérapeutique et cibler de manière sélective les lésions pour ne pas endommager les tissus sains, et une fluorescence suffisante afin de visualiser ces lésions au moyen de dispositifs médicaux.

RESUME DE L'INVENTION

Devant cette problématique, les inventeurs ont synthétisé à l'aide d'un procédé garantissant d'excellents rendements un nouveau photosensibilisateur comprenant un motif pyrophéophorbide-a conjugué avec l'acide folique *via* un bras espaceur de type polyéthylène glycol (Pyro-PEG-FA). L'analyse de ce photosensibilisateur a permis de mettre

en évidence des propriétés physicochimiques améliorées par rapport aux conjugués proches structurellement décrits ci-dessus ainsi qu'une bonne stabilité à la lumière. Les inventeurs ont également démontré que ce nouveau photosensibilisateur permettait de cibler spécifiquement les métastases péritonéales microscopiques des cancers épithéliaux de l'ovaire sans altérer la qualité de la réponse immunitaire effectrice antitumorale.

La présente invention concerne donc un composé de formule (I) :

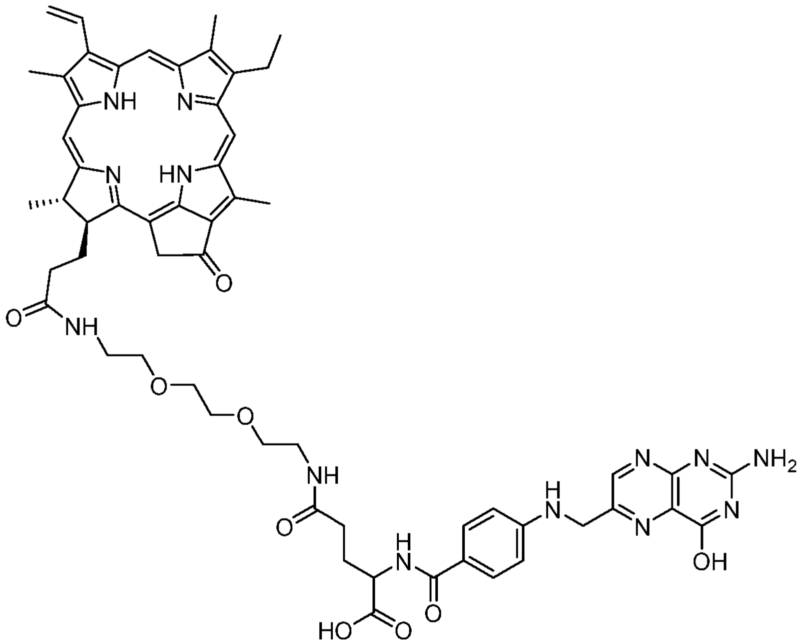

(I),

et ses sels pharmaceutiquement acceptables.

L'invention concerne également l'utilisation de ce composé de formule (I) en tant que médicament.

Selon un mode particulier de l'invention, le composé de formule (I) est utilisé pour le traitement d'un cancer, et notamment par thérapie photodynamique. Particulièrement, le composé de formule (I) de l'invention est utilisé pour réduire le risque de développer des métastases. De préférence, le composé de formule (I) est utilisé pour le traitement d'un cancer choisi parmi un cancer des ovaires, des poumons, du rein, de l'endomètre, colorectal, du sein, cancer du pancréas, du cerveau, gastrique, du foie, de la prostate, des testicules, de la vessie, ou de la tête et du cou. Plus préférentiellement, le composé de formule (I) est utilisé pour le traitement d'un cancer des ovaires.

Selon un autre mode particulier, le composé de formule (I) est destiné à être administré par voie intrapéritonéale ou par voie intraveineuse.

Un autre objet de l'invention concerne un procédé de préparation d'un composé de formule (I) tel que représenté ci-dessus comprenant une étape de couplage entre un composé de formule (II) :

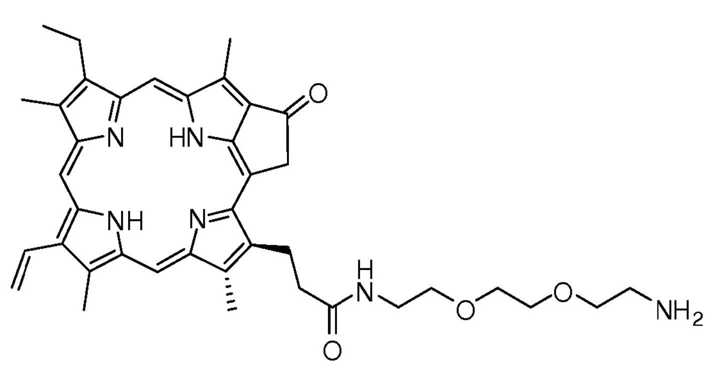

(II),

et l'acide folique.

Un autre objet de l'invention concerne également une utilisation du composé de formule (I) tel que représenté ci-dessus en tant que marqueur fluorescent.

Un autre objet de l'invention concerne aussi une méthode d'imagerie chez un sujet comprenant la visualisation

de la fluorescence émise par un composé de formule (I) préalablement administré audit sujet et photoactivé par une source lumineuse.

LEGENDE DES FIGURES

**Figure 1 :** Procédé de synthèse du composé Pyro-PEG-FA de formule (I).
**Figure 2 :** Photodégradation de l'acide folique (FA), du composé Pyro-PEG-FA de formule (I) (Pyro-S-FA), du composé Pheo-PEG-FA (Pheo-S-FA), et du composé TPP-FA (P1-S-FA) au cours du temps (365 nm, 5mW/$cm^2$, [0,45 mM] dans le DMSO).
**Figure 3A :** Mesure du taux relatif d'ATP dans les cellules tumorales ovariennes SKOV-3 (A) et OVCAR-3 (B) soumises à la PDT.
**Figure 3B :** Impact de la PDT sur la sécrétion de cytokines par les cellules tumorales ovariennes OVCAR-3.
**Figure 4A :** Photographie d'une image de coelioscopie chez un rat immunocompétent ayant développé une carcinose péritonéale après injection du composé Pyro-PEG-FA de formule (I).
**Figure 4B :** Photographie de la figure 4A en lumière blanche.

DESCRIPTION DETAILLEE DE L'INVENTION

Tel qu'illustré et démontré par les inventeurs dans les exemples ci-dessous, la présente invention fournit un nouveau photosensibilisateur couplé à un folate de formule (I) :

i) permettant de cibler spécifiquement les métastases péritonéales microscopiques des cancers épithéliaux de l'ovaire,
ii) ayant une bonne efficacité thérapeutique,
iii) offrant une fluorescence suffisante pour visualiser les lésions à l'aide de dispositifs médicaux conventionnels,
iv) nécessitant peu d'étapes pour sa synthèse, et
v) activant la prolifération des cellules immunitaires.

Composé Pyro-PEG-FA de formule (I)

La présente invention porte donc sur un composé de formule (I) :

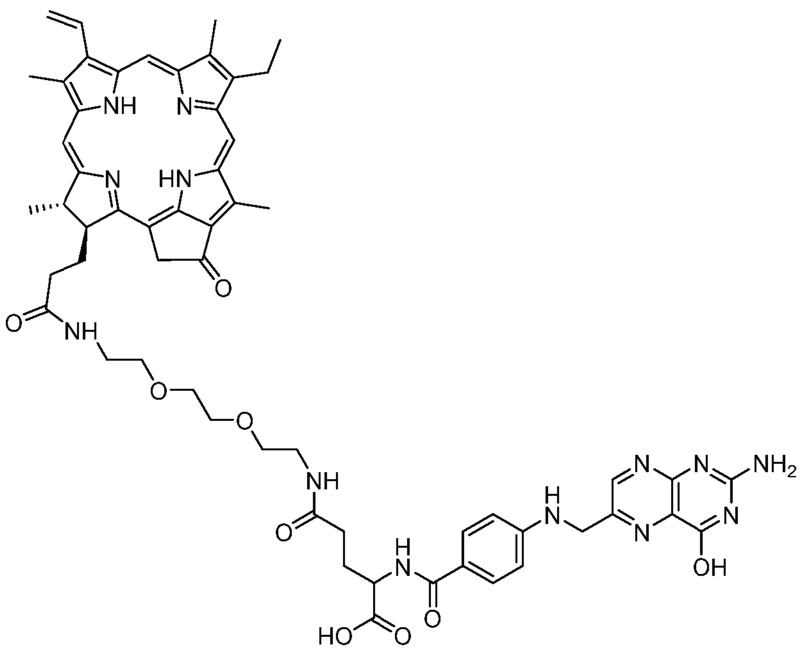

(I),

et ses sels pharmaceutiquement acceptables.

Ce composé de formule (I) est un composé conjugué comprenant un motif pyrophéophorbide-a conjugué avec

l'acide folique *via* un bras espaceur de type polyéthylène glycol (Pyro-PEG-FA). Le motif pyrophéophorbide-a apporte au composé de formule (I) une fluorescence satisfaisante pour visualiser les lésions. Le bras espaceur de type polyéthylène glycol comprend deux monomères de polyéthylène glycol $PEG_2$. Le motif acide folique permet de cibler spécifiquement les récepteurs de l'acide folique. Dans la présente description, le composé de formule (I) est représenté par « Pyro-PEG-FA » ou « Pyro-$PEG_2$-FA ».

L'expression "sel(s) pharmaceutiquement acceptable(s)" désigne les sels du composé d'intérêt de formule (I) qui possède l'activité biologique souhaitée. Les sels pharmaceutiquement acceptables comprennent des sels de groupes acides ou basiques présents dans le composé spécifié. Les sels d'addition acide pharmaceutiquement acceptables comprennent, mais ne sont pas limités à, des sels de chlorhydrate, bromhydrate, iodhydrate, nitrate, sulfate, bisulfate, phosphate, phosphate acide, isonicotinate, acétate, lactate, salicylate, citrate, tartrate, pantothénate, bitartrate, ascorbate, succinate, maléate, gentisinate, fumarate, gluconate, glucaronate, saccharate, formate, benzoate, glutamate, méthanesulfonate, éthanesulfonate, benzènesulfonate, ptoluènesulfonate et le pamoate (c'est-à-dire, 1,1'- méthylène-bis-(2-hydroxy-3-naphtoate)). Des sels de base adaptés comprennent, mais ne sont pas limités à, des sels d'aluminium, calcium, lithium, magnésium, potassium, sodium, zinc, et diéthanolamine. Une liste de sels pharmaceutiquement acceptables est notamment publiée dans la revue de Berge et al. (J. Pharm. Sci. 1977, 66(1), 1-19).

Le composé de formule (I) peut être synthétisé à l'aide d'un procédé simple comprenant une étape de couplage entre un composé de formule (II) :

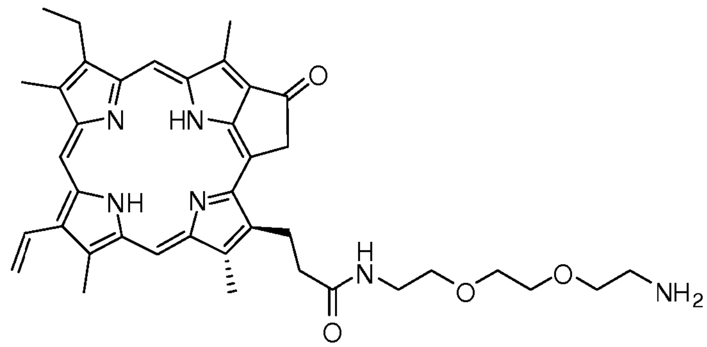

(II),

et l'acide folique.

Plus particulièrement, le composé de formule (I) peut être synthétisé à l'aide d'un procédé comprenant trois étapes à partir du pyrophéophorbide-a de formule (IV) tel que commercialisé par la société Boc sciences. Selon un mode de réalisation préféré, le composé de formule (I) est préparé par un procédé comprenant les étapes suivantes :

(a) une étape de couplage entre un composé de formule (IV) :

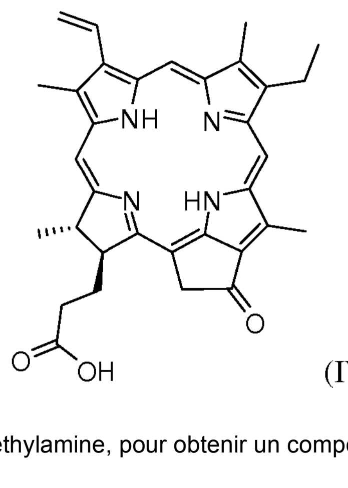

(IV),

avec du N-Boc-2,2'-(éthylènedioxy)diéthylamine, pour obtenir un composé de formule (III) :

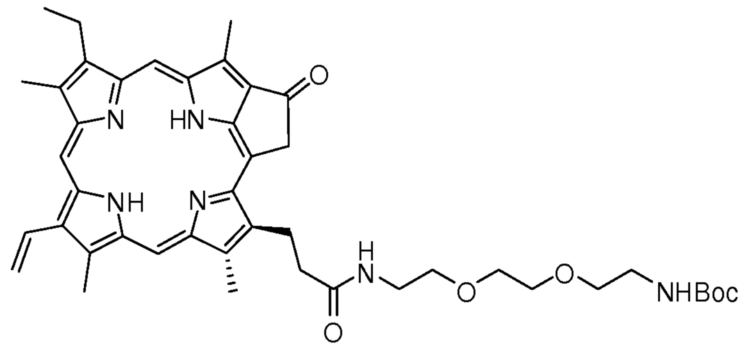

(III),

b) une étape de déprotection du composé de formule (III) obtenu à l'étape a) pour obtenir un composé de formule (II) :

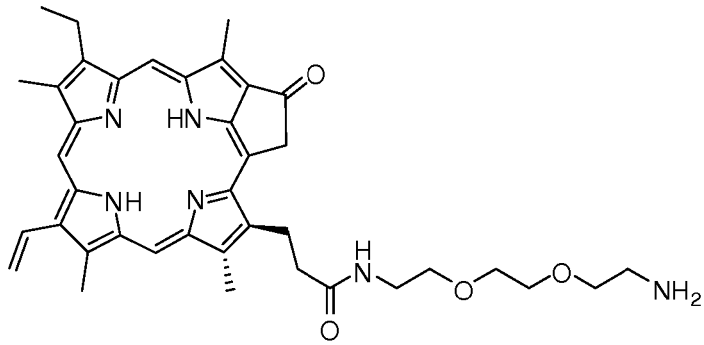

(II),

et

c) une étape de couplage entre le composé de formule (II) obtenu à l'étape b) et l'acide folique pour obtenir un composé de formule (I) :

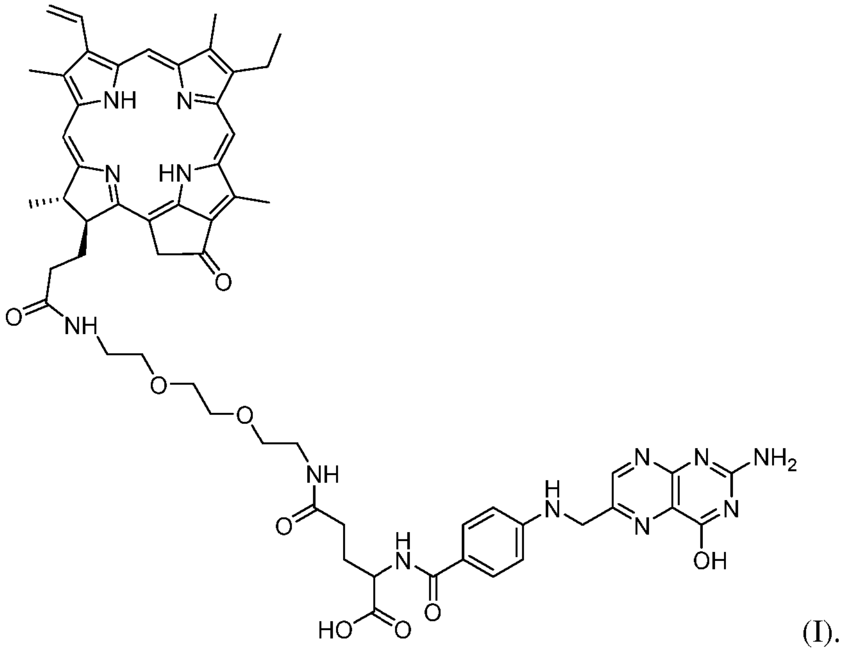

(I).

Ce procédé simple qui ne nécessite que trois étapes permet d'obtenir le composé de formule (I) avec des rendements très satisfaisants, qui sont notamment supérieurs par rapport aux rendements obtenus par le procédé mettant en œuvre le composé de You et al. (Pheo-PEG-FA). Ce procédé est donc plus avantageux et est en adéquation

avec une application industrielle.

Application

Le composé de formule (I) tel que décrit ci-dessus peut être utilisé en tant que médicament, de préférence dans le traitement du cancer.

Au sens de la présente invention, les termes « traitement » et « traiter » désignent une amélioration, la prophylaxie ou l'inversion d'une maladie ou d'un trouble, en l'occurrence un cancer. Par « traitement d'un cancer », on entend également la réduction du nombre des métastases et/ou la réduction du risque de développer des métastases. Dans le cadre du traitement des métastases, l'objectif est notamment de diminuer le taux de récidive ou de rechute pour le patient de développer un cancer. Par « traitement d'un cancer », on entend aussi l'inhibition de la prolifération des cellules cancéreuses.

Sans vouloir être lié à un mécanisme d'action particulier, le composé de formule (I) permet d'activer le système immunitaire, participant ainsi à la lutte contre le cancer. Ainsi, il est proposé d'utiliser le composé de formule (I) selon l'invention dans le traitement du cancer par immunothérapie, notamment par activation du système immunitaire après traitement photodynamique.

Plus particulièrement, le composé de formule (I) est utilisé dans le traitement du cancer par thérapie photodynamique.

La thérapie photodynamique, communément désignée par l'acronyme PDT consiste à mettre en contact un tissu pathologique avec une molécule photoactivable (nommée photosensibilisateur ou agent photosensibilisant), en l'occurrence le composé de formule (I) dans le cadre de la présente invention, puis à illuminer ce tissu avec une lumière de longueur d'onde (couleur) en adéquation avec les caractéristiques d'activation du photosensibilisateur. Après activation de la molécule par la lumière et réaction avec l'oxygène du tissu, des espèces très toxiques sont produites localement pour induire in fine la destruction de la lésion cancéreuse. L'avantage majeur de la PDT est sa sélectivité. En effet, la lumière utilisée, seule n'est pas nocive, le photosensibilisateur sans lumière n'est pas toxique. Pour induire la réaction, il faut une action conjointe de la lumière, du photosensibilisateur et de l'oxygène. Ainsi, en optimisant la concentration du photosensibilisateur et la dose de lumière, il est possible de détruire sélectivement des cellules.

Ainsi, l'invention concerne aussi un composé de formule (I) tel que décrit ci-dessus pour son utilisation dans le traitement du cancer, dans laquelle une dose efficace du composé de formule (I) est mise au contact des cellules cancéreuses et/ou des métastases qui sont ensuite exposées à une source lumineuse.

L'invention concerne également une méthode pour traiter un cancer comprenant les étapes de mise en contact des cellules cancéreuses avec une dose efficace du composé de formule (I) tel que décrit ci-dessus, et d'exposition des cellules cancéreuses à une source lumineuse.

Par « dose efficace », on entend une dose suffisante pour obtenir une fluorescence satisfaisante et obtenir ainsi l'effet thérapeutique désiré. L'homme du métier est capable d'adapter cette dose en fonction de la sévérité et du type de cancer à traiter. Néanmoins, une dose efficace du composé de formule (I) peut être comprise entre 0,1 mg/kg et 100 mg/kg, entre 0,1 mg/kg et 50 mg/kg, entre 0,1 mg/kg et 10 mg/kg, de préférence entre 0,1 mg/kg et 5 mg/kg, et de manière encore plus préférée entre 0,1 mg/kg et 3 mg/kg.

La photoactivation du photosensibilisateur, en l'occurrence le composé de formule (I) dans le contexte de la présente invention, peut être obtenue par toute sorte de source lumineuse connue de l'homme du métier. En particulier, la photoactivation peut être réalisée par lumière artificielle (lampes, lasers) avec des radiations telles que des radiations de type ultra-violet, infrarouge, ou par lumière visible ou naturelle. Le mode d'illumination à l'aide de dispositifs lumineux placés dans la cavité du corps humain adéquate est choisi par l'homme du métier en fonction du type de cancer à traiter. Par exemple, pour un cancer du foie, le dispositif lumineux est placé dans la cavité péritonéale. Pour un cancer des ovaires, le dispositif lumineux est placé dans la cavité pelvienne ; etc. Comme exemples non-limitatifs de dispositifs lumineux, on peut citer un ballon lumineux tel que décrit par Munck et al. (Photodiagnosis and Photodynamic Therapy, 2016, 16, 23-26) ou un textile lumineux (LEF) tel que décrit par Guyon et al. (Journal of Biomédical Optics, 2012, 17(3)).

La longueur d'onde de la lumière utilisée est choisie de manière à obtenir un effet photosensibilisateur le plus efficace. En particulier, une longueur d'onde comprise entre 300 et 800 nm, de préférence entre 400 et 700 nm, et de manière plus préférée autour de 668 nm est utilisée.

L'irradiation est généralement appliquée à une dose comprise entre 1 et 200 joules/cm$^2$, de préférence entre 1 et 150 joules/cm$^2$, et de manière encore plus préférée autour de 150 joules/cm$^2$. Sur des cellules, une dose comprise entre 1 et 10 joules/cm$^2$ est généralement appliquée.

De préférence, une source lumineuse avec une intensité lumineuse de 1 à 150 mW/cm$^2$, de 1 à 100 mW/cm$^2$, de 30 à 70 mW/cm$^2$, de préférence autour de 50 mW/ cm$^2$ est utilisée. Sur des cellules, une source lumineuse avec une intensité lumineuse de 1 à 10 mW/cm$^2$, ou encore mieux d'environ 5 mW/cm$^2$ est utilisée.

La photoactivation du photosensibilisateur et l'exposition des cellules cancéreuses à une source lumineuse peut être réalisée pendant une période allant de 1 minute à 3 heures, 10 minutes à 2 heures, 30 minutes à 90 minutes,

de préférence pendant une durée d'une heure ou 60 minutes.

L'invention décrite dans la présente demande est préférentiellement mise en œuvre chez les êtres humains.

Le composé de formule (I) présente un motif folate et peut donc être utilisé dans le cadre de traitement par thérapie photodynamique pour tout type de cancer exprimant des récepteurs folate. Une liste de ces cancers exprimant des récepteurs folate est décrite par Assaraf et al. (Drug résistance Updates, 2014, 17, 89-95) et inclue notamment le cancer des ovaires, des poumons, du rein, de l'endomètre, colorectal, du sein, cancer du pancréas, du cerveau, gastrique, du foie, de la prostate, des testicules, de la vessie, ou de la tête et du cou.

Selon un mode préféré de l'invention, le cancer est choisi parmi un cancer des ovaires, des poumons, du rein, de l'endomètre, colorectal, du sein, cancer du pancréas, du cerveau, gastrique, du foie, de la prostate, des testicules, de la vessie, ou de la tête et du cou. Selon un mode encore plus préféré, le cancer est un cancer des ovaires.

Le composé de formule (I) peut être administré au patient par tout type de voies connues de l'homme du métier en fonction du type de cancer à traiter. Par exemple, la voie intrapéritonéale ou la voie par intraveineuse peuvent être utilisées pour traiter le cancer des ovaires. Selon un mode préféré de l'invention, le composé de formule (I) est destiné à être administré par voie intrapéritonéale ou par voie intraveineuse, de préférence par voie intraveineuse.

Au vu de ses propriétés d'absorption et de fluorescence améliorées, le composé de formule (I) peut être utilisé en tant que marqueur fluorescent. Par exemple, le composé de formule (I) en tant que marqueur fluorescent peut être utilisé dans le traitement du cancer par chirurgie guidée par la fluorescence, et/ou dans des méthodes d'imagerie ou de diagnostic.

Il est ainsi décrit une méthode d'imagerie ou de diagnostic d'un cancer chez un sujet, comprenant l'administration et l'irradiation ou la photoactivation d'un composé de formule (I) audit sujet, et la visualisation de la fluorescence émise. Cette méthode permet de visualiser les récepteurs de l'acide folique qui sont surexprimés dans les cellules cancéreuses. Cette imagerie permet ainsi de délimiter la zone cancéreuse et, également, de guider un geste chirurgical de type ablation, le cas échéant.

Un objet de l'invention est donc une méthode d'imagerie chez un sujet comprenant la visualisation de la fluorescence émise par un composé de formule (I) préalablement administré audit sujet et photoactivé par une source lumineuse.

Au vu de ses mêmes propriétés, le composé de formule (I) peut aussi être utilisé en tant que photosensibilisateur dans des applications cosmétiques et dermatologiques telles que le photorajeunissement, le traitement de l'acné vulgaire ou le vieillissement cutané.

La présente invention porte donc également sur une utilisation non-thérapeutique du composé de formule (I) décrit ci-dessus en tant que photosensibilisateur.

D'autres aspects et avantages de l'invention apparaîtront à la lecture des exemples qui suivent, qui doivent être considérés comme illustratifs et non limitatifs.

## EXEMPLES

### Exemple 1 : Synthèse du conjugué Pyro-PEG-FA de formule (I) selon l'invention et du conjugué Pheo-PEG-FA de You et al.

Le procédé de synthèse du conjugué Pyro-PEG-FA comprenant trois étapes est décrit ci-dessous et est également illustré par la figure 1.

### Etape a) Synthèse de Pyro-PEG-NHBoc (Formule III)

100 mg de pyropheophorbide a (0,19 mmol), 46,4 mg de *N*-Boc-2,2'-(éthylènedioxy)diéthylamine (0,19 mmol), 71,7 mg (0,38 mmol) d'EDC et 30,5 mg (0,25 mmol) de DMAP ont été solubilisés dans 30 mL de THF. Le mélange réactionnel a été agité à température ambiante, sous atmosphère d'azote et dans l'obscurité pendant 24 heures. Le solvant a ensuite été évaporé. La purification du matériau brut a été réalisée sur colonne chromatographique de gel de silice avec 5% d'EtOH dans $CH_2Cl_2$. Le composé Pyro-PEG-NHBoc est obtenu sous la forme d'un solide vert foncé avec un rendement de **92 %** (132 mg). Rf = 0.27 ($CH_2Cl_2$/EtOH = 95/5, v/v). $^1H$ NMR (300 MHz, DMSO-d6): $\delta$ (ppm) = -2.01 (s, 2H, NH, Pyro(a)-COOH), 1.30 (s, 9H, Boc), 1.60 (t, 3H, $CH_3$, Pyro(a)-COOH), 1.79 (d, 3H, $CH_3$, Pyro(a)-COOH), 2.98 (q, 2H, $CH_2$), 3.15, 3.35, 3.59 (3 x s, 3H x 3, $CH_3$, Pyro(a)-COOH), 3.46 (q, 2H, $CH_2$), 3.60 (m, 8H, $CH_2$), 3.65 (m, 2H, $CH_2$, Pyro(a)-COOH), 4.31, 4.56 (2 x d, 1H x 2, CH, Pyro(a)-COOH), 5.16 (q, 2H, $CH_2$, Pyro(a)-COOH), 6.34, 6.40 (2 x d, 1H x 2, =$CH_2$, Pyro(a)-COOH), 6.66 (s, 1H, NH-spacer), 7.83 (t, 1H, NH-spacer), 8.19 (q, 1H, -CH=, Pyro(a)-COOH), 8.88, 9.38, 9.65 (3x s, 1H x 3, CH, Pyro(a)-COOH). HRMS (ESI+): m/z calc. Pour $C_{44}H_{56}N_6O_6$ [M+H]+ 765.4334; trouvée 765.4304.

**Etape b) Synthèse de Pyro-PEG-$NH_2$ (Formule II)**

Le composé Pyro-PEG-NHBoc a été solubilisé dans 2 mL de TFA. La solution a été agitée pendant 2 heures à température ambiante dans l'obscurité et sous azote puis lyophilisée afin d'éliminer le TFA. Le résidu coloré a été solubilisé dans du $CH_2Cl_2$ (10 mL), et du carbonate de potassium anhydre a été ajouté jusqu'à ce que la couleur change de bleu en vert. Après filtration, la phase organique a été concentrée. La purification du matériau brut a été réalisée sur colonne chromatographique de gel de silice avec $CH_2Cl_2$ / EtOH (de 90:10 à 50:50, v / v), donnant Pyro-PEG-$NH_2$ (103 mg, **90%**) sous la forme d'un solide vert. Rf = 0.17 ($CH_2Cl_2$/EtOH = 1:1, v/v). $^1H$ NMR (300 MHz, DMSO-d6): $\delta$ (ppm) = -1.94 (s, 2H, NH, Pyro(a)-COOH), 1.63 (t, 3H, $CH_3$, Pyro(a)-COOH), 1.79 (d, 3H, $CH_3$, Pyro(a)-COOH), 2.87 (t, 2H, $CH_2$), 3.17 (q, 2H, $CH_2$), 3.23-3.62 (3 x s, 3H x 3, $CH_3$, Pyro(a)-COOH), 3.50 (s, 8H, $CH_2$), 3.71 (m, 2H, $CH_2$, Pyro(a)-COOH), 4.30, 4.58 (2 x d, 1H x 2, CH, Pyro(a)-COOH), 5.17 (q, 2H, $CH_2$, Pyro(a)-COOH), 6.22, 6.39 (2 x d, 1H x 2, =$CH_2$, Pyro(a)-COOH), 7.98 (t, 1H, NH), 8.18 (s, 2H, $NH_2$), 8.24 (q, 1H, - CH=, Pyro(a)-COOH), 8.90, 9.45, 9.73 (3x s, 1H x 3, C20-10-5, Pyro(a)-COOH). HRMS (ESI+): m/z calc. pour $C_{39}H_{48}N_6O_4$ [M+H]+ 665.3810; trouvée 665.3801.

**Etape c) Synthèse de Pyro-PEG-FA (Formule I)**

De l'acide folique (0,95 eq. 63 mg) et du DCC (1 éq., 31 mg) ont été solubilisés dans un mélange de DMSO anhydre (5 mL) et de pyridine (2 mL). Le mélange a été agité pendant 15 min à température ambiante, à l'obscurité et sous azote. Pyro-PEG-$NH_2$ (0,9 éq.) a ensuite été ajouté et le mélange réactionnel a été agité pendant 24h à température ambiante. La solution a lentement été versée dans de l'éther diéthylique froid vigoureusement agité (45 mL). Par centrifugation, le précipité obtenu a été recueilli et lavé avec de l'éther diéthylique (50 mL). La poudre de Pyro-PEG-FA a été séchée sous vide (107 mg, **69%**). $^1H$ NMR (300 MHz, DMSO-d6): -1.97 (s, 2H, NH, Pyro(a)-COOH), 1.62 (t, 3H, C8 : $CH_3$, Pyro(a)-COOH), 1.76 (d, 3H, C18 : $CH_3$, Pyro(a)-COOH), 4.32 (2H, CH+C17, FA+Pyro(a)-COOH), 4.40 (d, 2H, $CH_2$, FA), 4.56 (d, 1H, C18, Pyro(a)-COOH), 5.17 (q, 2H, C13 : $CH_2$, Pyro(a)-COOH), 6.21, 6.50 (2 x d, 1H x 2, C3 : =$CH_2$, Pyro(a)-COOH), 6.60 (d, 2H, CHarom., FA), 7.29 (br, 3H, NH + $NH_2$), 7.61 (d, 2H, CHarom., FA), 7.81 (s, 2H, $NH_2$), 7.92 (d, 1H, NH, FA), 8.20 (q, 1H, C3 : -CH=, Pyro(a)-COOH), 8.59 (d, 1H, CHarom., FA), 8.88, 9.42, 9.68 (3x s, 1H x 3, C20-10-5, Pyro(a)-COOH). HRMS (ESI+): m/z calc. for $C_{58}H_{65}N_{13}O_9$ [M+Na]+ 1110.4920; trouvé 1110.4872.

Le conjugué de You et al. (Pheo-PEG-FA) a été synthétisé selon le même procédé de trois étapes décrit ci-dessus. Les rendements obtenus pour chacune des 3 étapes sont décrites dans le tableau 1 ci-dessous.

**Tableau 1 :**

| Composé | Prix | Rdt étape a) | Rdt étape b) | Rdt étape c) | Rdt Total |
|---|---|---|---|---|---|
| **Pheo-PEG-FA (You et al.)** | 1240€ / 1g (Inochem) | 63 % | 100 % | 49 % | **31 %** |
| **Pyro-PEG-FA (Formule I)** | 1280€/1g (Boc Sciences) | 92 % | 90 % | 69 % | **57 %** |

De manière surprenante, le procédé de synthèse décrit ci-dessus a permis d'obtenir le composé de formule (I) avec un rendement total deux fois supérieur par rapport au composé de You et al. La synthèse du composé de formule (I) est donc plus avantageuse au niveau industriel que le la synthèse du composé de You et al., notamment d'un point de vue économique.

**Exemple 2 : Propriétés photophysiques du conjugué Pyro-PEG-FA de formule (I) selon l'invention, du conjugué Pheo-PEG-FA de You et al., et du conjugué TPP-FA de Schneider et al.**

**Matériel et méthode**

Les spectres d'absorption ont été mesurés avec un spectrophotomètre UV-visible double faisceaux UV-3600 (SHIMADZU, MARNE LA VALLEE, France). Les spectres de fluorescence ont été mesurés avec un spectrofluorimètre Fluorolog FL3-222 (HORIBA Jobin Yvon, LONGJUMEAU, France) équipé d'une lampe à arc xénon de 450 W, d'un compartiment porte-cuve thermostaté (25°C), d'un photomultiplicateur UV-visible R928 (HAMAMATSU Japon) et d'un détecteur infrarouge InGaAs refroidi à l'azote liquide (DSS-16A020L Electro-Optical System Inc, Phoenixville, PA, USA). Le faisceau d'excitation est diffracté par un monochromateur double réseaux SPEX (1200 traits/mm blasé à 330 nm). La fluorescence a été mesurée par le détecteur UV-Visible via le monochromateur d'émission double réseaux SPEX (1200 traits/mm blasé à 500 nm). La production d'oxygène singulet a été mesurée par le détecteur infrarouge via le monochromateur d'émission double réseaux SPEX (600 traits/mm blasé à 1 $\mu$m) et un filtre passe haut à 780 nm. Tous les spectres ont été mesurés en utilisant des cuves quartz à 4 faces. Tous les spectres d'émission (fluorescence et luminescence de l'oxygène singulet) ont été rapportés à la même absorbance (inférieure à 0,2) avec les corrections de

lampe et de photomultiplicateur.

Le rendement quantique en fluorescence a été déterminé par l'équation (1) :

$$\phi_f = \phi_{f_0} \cdot \frac{I_f}{I_{f_0}} \cdot \frac{DO_0}{DO} \cdot \left(\frac{n}{n_0}\right)^2 \quad (1)$$

où $\Phi_f$ et $\Phi_{fo}$, If et $I_{fo}$, DO et $DO_o$, n et $n_o$ sont les rendements quantiques, les intensités de fluorescence, les densités optiques, les indices de réfraction de l'échantillon et de la référence respectivement.

La référence utilisée pour le calcul du rendement quantique de fluorescence est la tétraphénylporphyrine dans le toluène avec un $\varphi_{fo}$ de 0,11.

Le rendement quantique de production d'oxygène singulet est déterminé par l'équation (2) :

$$\phi_\Delta = \phi_{\Delta_0} \cdot \frac{I}{I_0} \cdot \frac{DO_0}{DO} \quad (2)$$

où $\Phi_\Delta$ et $\Phi_{\Delta o}$, I et $I_o$, DO et $DO_o$ sont les rendements quantiques de production d'oxygène singulet, les intensités de production d'oxygène singulet et les densités optiques de l'échantillon et de la référence respectivement.

La référence utilisée pour le calcul du rendement quantique de production d'oxygène singulet est le Rose de Bengale dans l'éthanol avec un $\varphi_{\Delta o}$ de 0,68

Les expériences de durées de vie de fluorescence ont été réalisées en utilisant pour l'excitation une diode laser pulsée émettant à 407 nm (LDH-P-C-400M, FWHM < 70 ps, 1 MHz) couplée à un générateur d'impulsions PDL 800-D (PicoQuant GmbH, BERLIN, Germany) et pour la détection une photodiode à avalanche SPCM-AQR-15 (EG & G, VAUDREUIL, Canada) couplée à un filtre passe haut à 650 nm.

L'acquisition a été réalisée par un module PicoHarp 300 associé à un routeur 4 voies PHR-800 (PicoQuant GmbH, BERLIN, Germany). Les déclins de fluorescence ont été enregistrés en utilisant la méthode de comptage monophotonique (Time Correlated Single Photon Counting TCSPC). Les données ont été collectées jusqu'à obtenir 1000 coups accumulés dans un canal puis analysés en utilisant le logiciel de TCSPC Fluofit (PicoQuant GmbH, BERLIN, Germany) basé sur une reconvolution itérative utilisant un algorithme de Levensberg-Marquandt permettant l'obtention de déclins multi-exponentiels.

Les mesures de durées de vie d'oxygène singulet ont été réalisées avec un spectrophotomètre TEMPRO-01 (HORIBA Jobin Yvon, LONGJUMEAU, France) composé d'une diode d'excitation pulsée SpectraLED-415 émettant à 415 nm, d'un compartiment porte-cuve, d'un monochromateur d'émission de type Seya-Namioka (600-2000 nm) et d'un tube photomultiplicateur proche infra-rouge régulé thermoélectriquement H10330-45 (HAMAMATSU, Japon). Le montage est piloté par le module de comptage FluoroHub-B et les logiciels DataStation et DAS6 (HORIBA Jobin Yvon).

Les spectres d'émission et les mesures de durées de vie de phosphorescence ont été réalisés avec un spectrofluorimètre Fluorolog FL3-22 (HORIBA Jobin Yvon, LONGJUMEAU, France) équipé d'une lampe xénon continue 450 W, d'une lampe flash xénon, d'un compartiment porte-cuve thermostaté (25°C) et d'un photomultiplicateur UV-visible R928 (HAMAMATSU Japon). Le montage est piloté par le module de comptage FluoroHub-B. Le logiciel FluorEssence (HORIBA Jobin Yvon) a été utilisé pour les spectres d'émission ; les logiciels DataStation et DAS6 (HORIBA Jobin Yvon) ont été utilisés pour la mesure des durées de vie de phosphorescence.

## Résultats

Les propriétés photophysiques dans l'éthanol sont indiquées dans le tableau 2 ci-dessous.

**Tableau 2 :**

| **Composé** | **$\varepsilon_{bande\ de\ soret}$ (L.mol$^{-1}$.cm$^{-1}$)** | **$\lambda_{QI}$ (nm)** | **$\varepsilon_{QI}$ (L.mol$^{-1}$.cm$^{-1}$)** | **$\phi_F$ (± 0,02)** | **$\phi_\Delta$ (± 0,05)** | **$\tau_F$ (± 0,1 ns)** | **$\tau_\Delta$ (± 1 $\mu$s)** |
|---|---|---|---|---|---|---|---|
| Pyro (a)-COOH | 76 326 | 668 | 33 265 | 0,34 | 0,52 | 6,7 | 13 |
| **Pyro-PEG-FA** | 74 081 | **668** | **35 306** | **0,30** | 0,54 | 6,4 | 13 |
| Pheo (a)-COOH | 69 663 | 667 | 32 537 | 0,44 | 0,52 | 5,7 | 12 |
| **Pheo-PEG-FA** | 49 215 | 667 | **22 870** | 0,40 | 0,48 | 5,7 | 13 |
| **TPP-FA** | 152 245 | **643** | 3 265 | **0,11** | 0,58 | 9,7 | 15 |
| $\varepsilon$ : coefficient d'extinction molaire ; Q : bande Q ; $\lambda_{exc}$ : longueur d'onde d'excitation ; $\phi_f$ : rendement quantique en fluorescence ; $\phi_\Delta$ : rendement quantique de luminescence ; $\tau_f$: durée de vie de fluorescence ; $\tau_\Delta$: durée de vie de l'oxygène singulet. | | | | | | | |

Le composé de l'invention Pyro-PEG-FA de formule (I) présente une absorption supérieure à celle du composé Pheo-PEG-FA de You et al. En particulier, le coefficient d'extinction molaire ($\varepsilon_{QI}$) à la longueur d'onde d'excitation utilisée en clinique (668 nm) est 1,5 fois supérieur.

Le composé de l'invention Pyro-PEG-FA de formule (I) présente une absorption supérieure à celle du composé TPP-FA de Schneider et al. En particulier, le composé de l'invention présente un pic d'absorption à 668 nm plus favorable à une meilleure pénétration que le composé TPP-FA (pic d'absorption à 643 nm). En outre, le rendement quantique de fluorescence ($\phi_F$) et le coefficient d'extinction molaire ($\varepsilon_{QI}$) sont respectivement environ 3 fois et 10 fois supérieur pour le composé de l'invention.

En conclusion, le composé de l'invention Pyro-PEG-FA de formule (I) présente des propriétés photophysiques améliorées en termes d'absorption par rapport aux composés Pheo-PEG-FA de You et al. et TPP-FA de Schneider et al.

**Exemple 3 : Stabilité à la lumière du conjugué Pyro-PEG-FA de formule** (I) **selon l'invention, du conjugué Pheo-PEG-FA de You et al., et du conjugué TPP-FA de Schneider et al.**

**Matériel et méthode**

Une solution de PS-bras-acide folique (Pyro-PEG-FA de formule (I)) (0,45 mM) dans le DMSO (3 mL) a été irradiée ($\lambda$ =365 nm, 5 mW.cm$^{-2}$, Xe-Hg Lamp, Lightningcure™ LC5 Hamamatsu) pendant 2 heures. 100 $\mu$L de solution ont été prélevés toutes les 15 minutes et dilués dans 1,9 mL de méthanol. Chaque échantillon (40 $\mu$L) a été analysé par chromatographie liquide haute performance en phase inverse (RP-HPLC) sur un appareil Prostar HPLC (Varian). La HPLC a été réalisée sur colonne Pursuit 5-$C_{18}$ (2,5 $\mu$m, 4,6 × 150 mm, Varian) en utilisant un gradient acétonitrile/(eau+0,1 % TFA) [10% : 90%] à [0% : 100%] en 25 minutes, suivi d'un plateau isocratique d'acétonitrile durant 10 minutes, puis retour aux conditions initiales en 5 minutes. Le pourcentage de dégradation a été calculé en comparant l'aire des pics correspondants au conjugué initial et au conjugué dégradé.

**Résultats**

Les résultats sont illustrés dans la figure 2.

Le composé de l'invention Pyro-PEG-FA de formule (I) est très largement plus stable que le composé Pheo-PEG-FA de You et al. et le composé TPP-FA de Schneider et al. En effet, après 2 heures d'irradiation (120 min), le composé Pyro-PEG-FA de l'invention présente 5 % de dégradation alors que les composés Pheo-PEG-FA et TPP-FA sont dégradés entre 30 et 40 %.

Au vu de sa bonne stabilité à la lumière, le composé de l'invention Pyro-PEG-FA de formule (I) est donc mieux adapté pour des applications en thérapie photodynamique.

## Exemple 4 : Evaluation biologique in vitro

### 4.1. Impact de la Thérapie Photodynamique sur des cellules tumorales et leur sécrétome

### Matériel et méthode

### I. Culture cellulaire

Les lignées tumorales ovariennes SKOV-3 et OVCAR-3 ont été achetées auprès de l'American Type Culture Collection (ATCC). Les SKOV-3 ont été mises en culture dans 50% DMEM (4,5 g/L D-Glucose, L-Glutamine ; Gibco) et 50% F-12 (Ham's F-12 Nutrient Mix, Gibco), les OVCAR-3 ont été mises en culture dans du RPMI-1640 contenant du L-Glutamine 2 mM 1%, 0,02 mM de sodium-pyruvate, 100 U/mL de pénicilline, 100 μg/mL de streptomycine et 10% de Sérum de veau foetal décomplémenté (Gibco).

### II. Thérapie photodynamique

Un million de cellules ont été déposées dans un flacon de 25 $cm^2$, après 24h, le milieu complet a été remplacé par un milieu contenant le composé de l'invention Pyro-PEG-FA de formule (I) (PS) (1 mg pour 100 mL de milieu). Au bout de 24h, le milieu contenant le PS a été remplacé par un milieu complet après deux rinçages au PBS (GIBCO BRL, Invitrogen, GB). Finalement, les cellules ont été soumises au laser d'une longueur d'onde spécifique (668 nm) durant une heure. 24h plus tard, le surnageant a été récupéré, centrifugé pour éliminer les cellules tumorales en suspension puis congelé à -20°C. 3 contrôles ont été utilisés : des cellules tumorales SKOV-3 et OVCAR-3 non traitées (NT), des cellules mises en contact avec le PS mais sans illumination (+PS), des cellules illuminées uniquement (+illu) et des cellules soumises à la PDT (PDT).

### III. Isolement des PBMCs

Les cellules mononuclées du sang périphérique (PBMCs) ont été isolées à partir de prélèvement sanguins dilué dans un volume de PBS stérile (GIBCO BRL, Invitrogen, GB) et déposées sur gradient de densité Ficoll-Paque TM Plus (Amersham Biosciences AB, Suède). Après une centrifugation de 40 min à 400 g sans décélération, l'anneau de cellules mononuclées a été récupéré, lavé 2 fois dans du PBS (300 g, 10 min) puis filtré.

### IV. PCR-Quantitative en Temps réel

#### 1. Extraction des ARNs

L'extraction des ARNs à partir des cellules NK, lymphocytes B, lymphocytes CD4+, lymphocytes T régulateurs (Treg) et les lymphocytes CD8+ a été réalisée à partir d'un culot sec de $2.10^5$ de cellules avec le kit d'extraction RNeasy Plus Mini Kit (QIAGEN, Hilden, Allemagne), selon les instructions du fabricant. 30 μL d'eau sans RNAse/DNase (UltraPure Distilled Water, Gibco BRL, Invitrogen, GB), ont été utilisés pour l'élution de l'ARN. L'extraction des ARNs SKOV-3, OVCAR-3 et PBMCs a été réalisée à partir d'un culot de $10^6$ cellules dissout dans 1 mL de Trizol (Invitrogen, Nouvelle-Zelande), selon les instructions du fabricant. Le culot a été solubilisé dans 10 μL d'eau sans RNAse/DNase. La quantification des ARNs a été réalisée par spectrophotométrie (Ultraspec 3100 Pro, Amersham Biosciences, USA). L'ARN total a été conservé à -80°C. Une électrophorèse en gel d'agarose 1% (UltraPure Agarose, Invitrogen, USA) permet de vérifier l'intégrité des ARNs extraits.

#### 2. Transcription inverse

Le kit de Transcriptase Reverse Superscript II a été utilisé pour la RT (GIBCO BRL, Invitrogen, GB). L'ADNc a été synthétisé à partir de 2 μg/μL d'ARN total, dans un volume de 15 μL d'eau. Puis, ont été ajoutés 5 μL du mélange suivant : 1 μL d'oligo dT (Roche, France) + 0,1 μL de RNAsin 40 U/μL (Promega, USA) + 4 μL d'eau sans RNAse/Dnase. Après 10 min à 70°C puis 5 min à TA, 10 μL d'un second mélange contenant : 6 μL de Tampon 5X (Tris-HCl, KCl, $MgCl_2$, Invitrogen, GB) + 1 μL de DiThioThréitol (Invitrogen, GB) + 2 μL de dNTPs 10mM (Amersham Biosciences, GB) + 0,1 μL de RNAsin (Promega, USA) + 1 μL de Transcriptase Reverse Superscript II (Invitrogen, GB) ont été ajoutés. L'échantillon a été incubé 1h à 45°C puis 5 min à 95°C. La réaction a été stoppée par l'ajout de 70 μL d'eau/μg d'ARN initial, la concentration finale était de 10 ng/μL.

### 3. PCR-Quantitative

Le principe est de suivre la néosynthèse du double brin d'ADN, grâce à la mesure de l'incorporation du Sybr-Green (fluorochrome intercalant l'ADN). Cette réaction a été réalisée à partir d'ADNc à une concentration équivalente à 10 ng d'ARN/μL de mélange réactionnel. Les amorces ont été conçues et synthétisés spécifiquement pour la Q-PCR (MWG-Biotech, Allemagne). Les résultats ont été normalisés par 3 gènes de ménage : 18S, GAPDH et HPRT (tableau 3 ci-dessous).

Les transcrits ont été quantifiés par le Mx3005P (Stratagène, USA) dans des plaques optiques 96 puits (Eurogentech, France). Dans chaque puits, 10 μL d'un couple d'amorces spécifiques (10 pmol/μL) ont été déposés, chaque plaque contenant les 44 couples amorces et un puits contrôle contenant de l'$H_2O$, en double exemplaire. Les Q-PCR ont été réalisées, à partir de 1 μL d'échantillon d'ADNc (concentration équivalente à 10 ng d'ARN/μL)/puits, suivant les instructions du fabricant avec le kit MESA GREEN qPCR MasterMix Plus for SYBR® Assay (Eurogentec, France). Après une première dénaturation de 5 minutes à 95°C, le mélange réactionnel a subi 45 cycles d'amplification consistant en une succession de passage de 15s à 95°C (Dénaturation du double brin d'ADN) puis de 1 minute à 60°C (Hybridation des amorces et élongation du brin d'ADN néosynthétisé). L'intensité de fluorescence a été mesurée à la fin de chaque cycle d'élongation et un cycle de fusion a été programmé immédiatement après la dernière amplification. L'expression simultanée des deux isoformes du récepteur aux Folates, FOLR1 et FOLR2, par les cellules tumorales SKOV-3 et OVCAR-3 a été analysée mais aussi par différentes cellules immunitaires telles que les PBMCs, les lymphocytes Natural Killer (NK), les lymphocytes B, les lymphotes CD4+, les lymphocytes CD8+ et les lymphocytes T régulateurs.

### 4. Analyse et représentation des résultats

L'expression quantitative d'un gène a été interprétée en utilisant la méthode ΔCT. L'expression des gènes est donnée en « CT » (Cycle Threshold) puis normalisée par la moyenne des 3 gènes de ménages = ΔCT. Les expériences ont été réalisées en duplicata.

**Tableau 3 : Récapitulatif des amorces**

| | Primer sens et anti-sens | |
|---|---|---|
| **FOLR1** | 5'-AGGTGCCATCTCTCCACAGT | 5'-GAGGACAAGTTGCATGAGCA |
| **FOLR2** | 5'-CTGGCTCCTTGGCTGAGTTC | 5'-GCCCAGCCTGGTTATCCA |
| **18S** | 5'-TCAAGAACGAAAGTCGGAGG | 5'-GGACATCTAAGGGCATCACA |
| **GAPDH** | 5'-GCCAAGGTCATCCATGACAACTTTGG | 5'-GCCTGCTTCACCACCTTCTTGATGTC |
| **HPRT** | 5'-CCCTGGCGTCGTGATTAG | 5'-ATGGCCTCCCATCTCCTT |

### V. Test de viabilité

***Sur les cellules tumorales*** : 2000 cellules tumorales (SKOV-3 et OVCAR-3) ont été mises en plaque 96 puits à fond blanc (Falcon®) dans 100 μL de leur milieu correspondant (DMEM/F-12 et RPMI). Les cellules ont été activées par 1μg/mL d'anticorps (Ac) anti-CD3, préalablement appliqués sur les plaques (2h à 37°C), et 1 μg/mL d'anticorps anti-CD28. La mise en culture est réalisée selon une cinétique de 3h, 24h, 72h et 120h puis un test de viabilité a été réalisé pour chaque temps. Ce test permet la quantification d'ATP présente dans les cellules grâce à la réaction de luciférase pour déterminer le nombre de cellules viables en culture.

***Sur les cellules immunitaires*** : $10^5$ PBMCs ont été mises en culture avec 50% de ML10 (50 μL) et 50% du surnageant des cellules tumorales, soumises aux différentes conditions (Non traitées, PS uniquement, illumination uniquement et soumises à la PDT), préalablement recueilli (50 μL).

### VI. Test de prolifération

$10^5$ de PBMCs ont été cultivées en plaques 96 puits à fonds ronds (BD Falcon®), dans 50 μL de milieu de culture : RPMI-1640, L-Glutamine 2 mM 1%, 0,02 mM de sodium-pyruvate, 100 U/mL de pénicilline, 100 μg/mL de streptomycine, 10% de Sérum Humain AB décomplémenté (GIBCO BRL, Invitrogen, GB) et 50 μL du surnageant des

cellules tumorales selon les différentes conditions (NT, PS uniquement, illumination uniquement et PDT). Les cellules ont été activées par 1 μg/mL d'anti-CD3, préalablement déposés sur les plaques (2h à 37°C), et 100 ng/mL d'anti-CD28. La prolifération a été évaluée par incorporation de 1 μCi/puits de Thymidine Tritiée ($^3$H-Th) (GE HEALTHCARE, France), 18h avant la fin de la culture. Selon une cinétique de 24h, 48h, 72h et 120h, les plaques ont été filtrées et les filtres ont été lus dans un compteur de radioactivité (1450 Trilux, Wallac, Finlande). Les expériences ont été réalisées en *triplicata* et les résultats sont exprimés en coups par minute (cpm).

**VII. Cytométrie en Flux**

Les anticorps monoclonaux anti-humains utilisés ont été couplés à des fluorochromes (Voir tableau 4 ci-après). Pour le contrôle, un isotype des différents anticorps monoclonaux a été utilisé en tant que contrôle. $10^5$ cellules ont été reprises dans un volume de 100 μL de PBS stérile et incubées avec 5 μL de chaque Ac (10 min à T° ambiante (TA) et à l'obscurité). Les cellules marquées ont été lavées par ajout de 200 μL de PBS puis centrifugation 5 minutes à 600 g, le surnageant a ensuite été jeté. Les cellules ont ensuite été reprises dans 200 μL de PBS et enfin analysées par cytométrie de flux (BD FACSCanto -II).

**Tableau 4 : Représentation des différents anticorps monoclonaux anti-humains utilisés pour la cytométrie en flux.**

| Panel | **Anticorps** | **Isotypes** |
|---|---|---|
| **Type cellulaire** | CD4 (VIT4)-VioBlue | IgG2a (de souris)-VioBlue |
| | CD8-VioGreen | IgG2a (de souris)-VioGreen |
| | CD19-VioBright FITC | IgG1 (de souris)-VioBright FITC |
| | CD14-PE | IgG2a (de souris)-PE |
| | CD3-PE-Vio770 | IgG2a (de souris)-PE-Vio770 |
| | CD335 (NKp46)-APC | IgG1 (de souris)-APC |
| | CD11C-APC-Vio770 | IgG2b (de souris)-APC-Vio770 |
| **Activation** | CD4 (VIT4)-VioBlue | IgG2a (de souris)-VioBlue |
| | CD30-APC-Vio770 | IgG1 (de souris)-APC-Vio770 |
| | CD69-PE-Vio770 | IgG1 (de souris)-PE-Vio770 |
| | Anti-HLA-DR-PerCP-Vio700 | IgG2a (de souris)-PerCP-Vio700 |
| | CD152-APC | IgG2a (de souris)-APC |
| | CD197 | REA Contrôle (S)-PE |
| | CD25-VioBright FITC | IgG2b (de souris)-VioBright FITC |
| | CD8-VioGreen | IgG2a (de souris)-VioGreen |
| **Treg naturels** | CD4 (VIT4)-VioBlue | IgG2a (de souris)-VioBlue |
| | CD25-VioBright FITC | IgG2b (de souris)-VioBright FITC |
| | CD127-PE-Vio770 | IgG2a (de souris)-PE-Vio770 |
| **Treg induits** | CD4 (VIT4)-VioBlue | IgG2a (de souris)-VioBlue |
| | CD18-FITC | IgG1 (de souris)-FITC |
| | CD223-PE | REA Contrôle (S)-PE |
| | CD49b-PE-Vio770 | REA Contrôle (S)-PE-Vio 770 |
| | CD152-APC | IgG2a (de souris)-APC |

**Résultats**

L'analyse transcriptomique a montré que les lignées de cellules tumorales ovariennes étudiées (SKOV-3 et

OVCAR-3) exprimaient préférentiellement l'isoforme FOLR1 montrant ainsi leur potentielle sensibilité vis-à-vis du nouveau photosensibilisateur de l'invention Pyro-PEG-FA de formule (I) et donc leur sensibilité à la mort induite par la thérapie photodynamique (PDT).

A l'issue de la PDT, l'analyse morphologique in vitro des cellules tumorales ovariennes SKOV-3 et OVCAR-3 a montré qu'elles étaient sensibles à la PDT avec un effet visible dès 1 heure post-illumination (cellules non adhérentes, aspect de lyse cellulaire). Ces résultats ont été confortés par un test de viabilité (MTT) sur les cellules tumorales ovariennes soumises in vitro à la thérapie photodynamique. En effet, il a été observé que les cellules tumorales ovariennes SKOV-3 et les OVCAR-3 soumises à la PDT présentaient une diminution importante de leur viabilité cellulaire au cours du temps. Par contre, il a été observé qu'il n'y avait pas de modification notable de la viabilité des cellules tumorales non traitées ou soumises uniquement au PS ou à l'illumination. Ainsi, ces résultats valident l'efficacité du PS de l'invention Pyro-PEG-FA de formule (I) et montrent que la PDT est bien capable d'induire la mort des cellules tumorales ovariennes avec un effet très rapide, puisque 90% des cellules tumorales sont mortes après seulement 1 heure d'illumination (Figure 3A).

L'impact du sécrétome des cellules tumorales ovariennes soumises à la PDT sur les cellules mononucléées du sang périphérique humaines (PBMC) a été étudié. Les PBMC, mis en culture avec du surnageant de OVCAR-3 ou de SKOV-3 soumises à la PDT, ont montré une augmentation de leur viabilité après seulement 3 heures de mise en culture. Cette augmentation du métabolisme mitochondrial a été obtenue dans 3 expériences indépendantes et est statistiquement significative (à 48 h et 120 h). Ces résultats illustrent donc que la PDT utilisant ce nouveau photosensibilisateur Pyro-PEG-FA de formule (I) peut modifier le sécrétome des cellules tumorales en faveur d'un sécrétome activant la prolifération des cellules immunitaires humaines.

L'effet du sécrétome des cellules tumorales SKOV-3 soumises à la PDT sur le phénotype des populations immunitaires a été analysé. Pour cela, l'expression des marqueurs des différentes populations immunitaires a été analysée par cytométrie en flux. Les résultats obtenus ont montré que le sécrétome des cellules tumorales SKOV-3 n'induisait pas de modification du pourcentage des lymphocytes T CD4+ et T CD8+, des monocytes, des lymphocytes B ou des NK dans les PBMC, quel que soit les conditions de cultures. Par ailleurs, les marqueurs d'activation précoces et tardifs ont été spécifiquement analysés dans les T CD4+, et les résultats ont montré que le sécrétome des cellules tumorales ovariennes soumises aux différents traitements n'induisait pas non plus de modification de l'activation des T CD4+. Ainsi, contrairement à la radiothérapie ou la chimiothérapie, l'ensemble des résultats obtenu a montré que la PDT utilisant le nouveau photosensibilisateur Pyro-PEG-FA de formule (I) n'altérait pas la qualité de réponse immunitaire effectrice anti-tumorale.

L'impact du sécrétome des cellules SKOV-3 soumises à la PDT sur les populations de lymphocytes T régulateurs naturels et induits (Tr1) a également été étudié. L'analyse des résultats obtenus (en analysant les Tr1 sur la base des triples marquages CD4+CD18+CD49b ou CD4+CD49b+LAG3+) a montré que le sécrétome des cellules SKOV-3 non traitées induit des populations de type Tr1. De manière surprenante, il a été observé que les différents traitements (Illumination+, PS+ ou PDT) ne favorisent pas cette induction de Tr1. Ainsi, contrairement à la radiothérapie classique, la PDT utilisant ce nouveau photosensibilisateur Pyro-PEG-FA de formule (I) ne modifie pas le sécrétome des cellules tumorales en faveur d'un environnement encore plus immunosuppressif qui serait favorable à l'échappement de la tumeur au système immunitaire et donc à la progression tumorale.

### 4.2.Activation des cellules immunitaires

L'état d'activation des différentes populations lymphocytaires T notamment les lymphocytes T (LT) CD4+ (CCR7+, CD25, CD30, CD69, CTLA4 et HLADR) et les LT CD8+ (CCR7+, CD25, CD30, CD69, CTLA4 et HLADR) a été analysé par cytométrie de flux.

On observe tout d'abord pour les LT CD4+ que le marqueur CD69+ (marqueur d'activation précoce) est présent de 24 heures jusqu'à 72 heures. La PDT permet une expression du marqueur CD69+ plus importante que les cellules qui n'ont pas été traitées. Pour ce qui est des conditions illumination seule ou photosensibisateur (Ps) seul, les résultats ne montrent pas de variation majeure par rapport aux cellules non traitées. Pour les LT CD8+, le marqueur CD69+ est présent de 24 heures jusqu'à 120 heures. Les cellules soumises à la PDT permettent une augmentation de l'expression du marqueur CD69+, qui est marqueur d'activation précoce, puisque la médiane de fluorescence est plus grande que pour les cellules non traitées. Pour ce qui est des conditions ILL seule ou Ps seul, les résultats ne montrent pas de variation majeure par rapport aux cellules non traitées.

Ainsi, ces résultats suggèrent que les cellules de carcinoses ovariennes soumises à la PDT, produisent des facteurs favorisant l'activation précoces des lymphocytes T CD4+ et CD8+.

On observe une présence du marqueur CTLA4+ (marqueur d'activation précoce) pour les LT CD4+ et CD8+ de 24 heures à 72 heures. On remarque que la médiane de fluorescence est plus importante dans les deux cas pour les cellules soumises à la PDT par rapport aux cellules non traitées. Ceci indique que les milieux conditionnés des cellules cancéreuses soumises à la PDT permettent de potentialiser une expression du marqueur sur les LT CD4+ et

les LT CD8+.

La condition illuminée permet une forte augmentation de l'expression de ce marqueur. Pour ce qui concerne la condition Ps seul on observe que l'expression du marqueur CTLA4+ est toujours plus faible que les cellules non traitées. Au bout de 72 heures, avec la condition PDT, on perd l'expression du marqueur CTLA4+, qui est remplacé par le marqueur HLADR+ (marqueur d'activation lié à la présentation d'antigène) à la fois pour les LT CD4+ et LT CD8+. Ainsi, nos résultats suggèrent que la PDT favorise indirectement l'expression (i) du HLADR, qui est favorable à l'activation immunitaire et (ii) du CTLA4 dans un premier temps, puis on observe une diminution de l'expression de ce marqueur. Ce résultat est cohérent puisque le CTLA4 est considéré plus tardivement au cours de la réponse immunitaire comme un immune check point, c'est-à-dire comme un point de rétrocontrôle négatif de la réponse immunitaire.

On observe à 24 heures, une augmentation de l'expression du marqueur CD30+ (marqueur d'activation tardif) essentiellement pour les cellules soumises à la PDT, c'est le cas pour le LT CD4+ à 48 heures. En revanche pour les LT CD8+, la condition PDT permet une surexpression du marqueur CD30+, qui est ensuite suivie d'une surexpression du marqueur CD25+ (marqueur d'activation et récepteur R-alpha de l'IL-2). Au bout de 72 heures le marqueur d'activation CD30+ diminue sur les LT CD4+ en condition PDT/condition non-traitées. En revanche pour les LT CD8+ l'expression du marqueur CD30+ est très importante en condition PDT. Au bout de 120 heures l'expression du marqueur CD25+ est augmentée dans les deux cas LT CD4+ et LT CD8+ pour les cellules en condition PDT. Ainsi, l'ensemble de ces résultats suggèrent que les cellules de carcinoses ovariennes soumises à la PDT, produisent des facteurs favorisant l'activation des lymphocytes T CD4+ et CD8+.

Après 48h et jusqu'à 120 heures de culture, on observe une augmentation de l'expression du marqueur CCR7+ (récepteur des chimiokines CCL19 et CCL21) à la fois pour LT CD4+ et LT CD8+. L'expression du marqueur est en générale plus importante pour les cellules soumises à la PDT. La PDT permet donc l'expression du marqueur CCR7+ qui est un récepteur de chimiokine impliqué dans la migration ganglionnaire des cellules immunitaires. A noter, que l'illumination seule permet elle aussi une augmentation importante de l'expression de ce marqueur CCR7+. Ainsi, ces résultats suggèrent que les cellules de carcinoses ovariennes soumises à la PDT, produisent des facteurs susceptibles de favoriser la migration des lymphocytes T CD4+ et CD8+ via les chimiokines CCL19 et 21.

Les inventeurs ont ainsi montré, par cytométrie de flux, que les milieux conditionnés induisent non seulement une expansion clonale des cellules immunitaires mais également une augmentation d'un certain nombre de marqueurs d'activation au cours du temps (précoces ou tardifs), suggérant que les cellules immunitaires sont effectrices, à savoir que les cellules immunitaires présentent un phénotype activé permettant d'assurer un effet anti-tumoral.

### 4.3.Impact sur la sécrétion de cytokines

Des immunoessais ont été réalisés sur le surnageant des cellules tumorales de carcinoses ovariennes OVCAR-3 traitées par la PDT afin de déterminer si les surnageants des cellules tumorales expriment et libèrent des cytokines après le traitement à la PDT.

Méthode

Un test ELISA sandwich a été réalisé. Dans un premier temps, l'anticorps primaire spécifique à la cytokine recherchée est fixé sur un support pendant tout une nuit à 4°C. Le lendemain un lavage au PBS-Tween 0,05% permet d'éliminer les anticorps non fixés. Afin d'éviter l'absorption d'autres molécules non spécifiques sur le support, on réalise une saturation en tapissant les zones libres avec une molécule (ici la bovine sérum albumin (BSA)) n'ayant aucune affinité avec les zones variables et spécifiques de l'anticorps primaire. Un lavage est ensuite réalisé afin d'éliminer les molécules en suspension, avant ajout de l'échantillon. Si la cytokine recherchée est présente, il y a formation d'un complexe immun avec l'anticorps primaire qui lui est spécifique. Les éventuelles molécules non spécifiques présentes restent en suspension. Un lavage permet aux éléments non fixés d'être éliminés. La dernière étape consiste à ajouter l'anticorps secondaire spécifique à la cytokine. La révélation s'effectue grâce à une enzyme, la peroxydase qui transforme un substrat chromogène (ortho-phenylènediamine) en un produit chromophore quantifiable au spectrophotomètre. Cette enzyme est couplée à la streptavidine. Grâce à la biotine présente sur l'anticorps secondaire qui possédé une forte affinité à la streptavidine, il y a formation du complexe biotine/streptavidine/peroxydase permettant la quantification. Pour arrêter la réaction enzymatique on ajoute un diacide (ici HCl).

Résultats

Les résultats sont présentés à la Figure 3B.

Interleukine 6 (IL6)

IL6 est une cytokine pro-inflammatoire c'est-à-dire quelle est capable de favoriser les processus inflammatoires ce qui n'est pas bénéfique puisque dans ce cancer en particulier, la production d'inflammation favorise la progression tumorale et la dissémination de de métastase. Les résultats montrent que pour les cellules Ovcar-3 contrôles non traitées (NT), on observe une concentration en cytokine IL6 de 53,45 pg/mL ce qui est une quantité importante. Pour les cellules Ovcar-3 illuminées on observe une forte diminution de la cytokine (9,06 pg/mL) par rapport au cellules non traitées ce qui permet de déduire que l'illumination des cellules cancéreuses ovarienne Ovcar-3 soumises à illumination d'une heure entraine la diminution de la présence des cytokine IL6 ce qui est très encourageant. Le Ps seul permet une diminution (29,08pg/mL) de la cytokine IL6. Pour les cellules Ovcar-3 test qui ont été traitées par la PDT, on observe une diminution (19,12 pg/mL) par rapport aux cellules non traitées (53,45 pg/mL).

Ainsi, ces résultats suggèrent que les cellules Ovcar-3 traitées par PDT diminuent leur production de cytokine IL6. Ces premiers résultats sont très favorables quant à l'utilisation de la PDT comme traitement anti-tumoral.

Interleukine 2 (IL2)

L'IL2 joue un rôle majeur dans l'homéostasie de la réponse immunitaire, en favorisant la survie et la prolifération notamment des lymphocytes T. Pour la condition contrôle cellule Ovcar-3 non traitées (NT), on détecte une présence de IL2 avec une concentration de 72,50 pg/mL. En revanche pour les conditions ILL et Ps on n'observe plus de cytokine IL2, avec une concentration égale à 0 pg/mL. Les cellules traitées par illumination seule et les cellules en contact avec le Ps seul provoquent donc une diminution importante de la sécrétion de la cytokine IL2. Pour les cellules Ovcar-3 soumises à la PDT, les inventeurs ont observé une augmentation de la production de l'IL2 (122,50 pg/mL) par rapport aux cellules non traitées ce qui nous montre que le traitement par la PDT est en faveur de la prolifération des LT.

Ainsi, ces résultats montrent que la PDT favorise la sécrétion de l'IL-2 par les cellules de carcinoses ovariennes, ce qui est en faveur d'un effet prolifératif sur les cellules immunitaires et sur les LT en particulier. Ce résultat est en corrélation avec l'augmentation de l'expression du CD25 sur les LT CD4+ et CD8+ (voir résultats de cytométrie).

Interféron gamma (IFN-gamma)

Pour la condition contrôle, avec les cellules cancéreuses non traitées, on détecte une concentration de 2,72 pg/mL d'IFN-gamma. Les cellules Ovcar-3 traitées par illumination permettent une très légère augmentation (2,85 pg/mL) de cette cytokine. En revanche pour les cellules Ovcar-3 en contact avec le Ps seul, l'IFN-gamma n'est pas détectable, ce qui veut dire que le Ps seul entraine l'élimination de cette cytokine. Pour la condition test (cellules soumises à la PDT), on observe une forte augmentation (28,55 pg/mL) d'IFN-gamma.

Ces résultats indiquent que la PDT est susceptible de favoriser la production d'IFN-gamma par les cellules de carcinoses ovariennes et donc de participer à un effet anti-tumoral via cette cytokine. En effet, l'IFN-gamma permet d'empêcher le développement des tumeurs malignes et des métastases notamment en inhibant l'angiogenèse, en stimulant la maturation des lymphocytes B et T, mais aussi en activant d'autres types de cellules immunitaires tel que les monocytes.

TGF-beta

Les cellules cancéreuses non traitées produisent le TGF-beta à une concentration de 28,94 pg/mL. La condition « cellules Ovcar-3 traitées par illumination » montre une diminution de la concentration en cytokine (8,95 pg/mL) ce qui est encourageant. En revanche pour les cellules Ovcar-3 en contact avec seulement le PS on observe une augmentation importante de la concentration en cytokine (49,56 pg/mL). Pour la condition PDT, on observe que la concentration a littéralement chutée (1,36 pg/mL).

Ainsi, les résultats obtenus suggèrent que la PDT diminue la production de la cytokine immunosuppressive TGF-beta par les cellules de carcinoses ovariennes. Cet effet est bénéfique puisqu'il permet de limiter le microenvironnement immunosuppressif qui est favorable à la croissance tumorale.

CONCLUSION

Les inventeurs ont étudié l'impact du surnageant des cellules cancéreuses de l'ovaire soumises à la PDT, sur les cellules mononucléaires du sang périphérique. Ils ont étudié l'état d'activation des différentes populations lymphocytaires T notamment les LT CD4+et les LT CD8+. Les résultats ont montré que les cellules Ovcar-3 soumises à la PDT sont capables d'activer les cellules lymphocytaires T CD4+ et T CD8+. Une augmentation de marqueurs d'activations CD69+, CTLA4+, CCR7+, CD30+, CD25+ et HLADR+ a ainsi été observée.

Les inventeurs ont également cherché la potentielle production de cytokines après traitement des cellules Ovcar-3 par PDT. Les résultats observés montrent que les cellules tumorales soumise à la PDT permettent une diminution de cytokines pro-inflammatoires telle que IL6 et une diminution de la présence de TGF-beta qui est immunosuppressive. Les résultats ont permis aussi de révéler une augmentation de cytokines favorables à la survie et la prolifération et l'activation des cellules immunitaires, telles que les cytokines IL2 et IFN-gamma.

Les produits de la PDT des cellules tumorales ovarienne favorisent la voie Th1 (voie de l'immunité cellulaire) notamment favorable à la réponse immunitaire anti-tumorale. De plus, ils n'activent pas les voies inflammatoires ou régulatrices.

**Exemple 5 : Etude In vivo**

**I. Carcinome ovarien de rat : Lignée cellulaire NuTu-19**

La lignée cellulaire NuTu-19 était une lignée d'adénocarcinome ovarien syngénique de rat permettant le développement de tumeur ovarienne chez un rat immunocompétent (Rose et al., Am. J. Obstet. Gynecol. 1996, 175(3 Pt 1), 593-9). Les cellules ont été conservées dans l'azote liquide ($5.10^6$ cellules par cryotube) puis cultivées dans du DMEM (*Gibco - Life Technologies*™) supplémenté avec 10% de sérum de veau fœtal décomplémenté, et 1% de mélange pénicilline / streptomycine et 1% de Glutamax. Les cellules ont été incubées dans des conditions standardisées ($CO_2$ 5%, humidité 100%, 37°C). Lorsque les cellules ont été à confluence, elles ont été trypsinisées afin de lever l'adhérence et permettre leur recueil (solution trypsine 0,25%, *Gibco - Life Technologies*™), lavées au PBS (Dulbecco's phosphate buffered saline, *Gibco - Life Technologies*™) et comptées après test d'exclusion au bleu trypan pour évaluer la viabilité. Les cellules ont été injectées sous forme de suspension cellulaire dans du PBS par voie intrapéritonéale aux différents rats afin d'obtenir un modèle de carcinose péritonéale.

**II. Modèle animal de carcinose péritonéale**

Nous avons utilisé un modèle validé de carcinose péritonéale (Rose et al., Am. J. Obstet. Gynecol. 1996, 175(3 Pt 1), 593-9). Les rats femelles Fisher F344 ont été obtenues auprès du fournisseur *Harlan*®. Les animaux hébergés à l'animalerie du Département Hospitalo-Universitaire de Recherche Expérimentale (DHURE - Université de médecine Pôle Recherche - CHRU de Lille). L'alimentation (*SAFE*™) et l'eau ont été fournies *ad libitum.*

Le modèle de carcinose a été obtenu par inoculation intrapéritonéale d'une suspension cellulaire de NuTu-19 dans du PBS ($20.10^6$ cellules par rat). Les rats ont été surveillés régulièrement jusqu'au développement d'une ascite témoignant de l'évolution tumorale, ou jusqu'à la réalisation d'une cœlioscopie exploratrice destinée à confirmer la présence de lésions de carcinose péritonéale.

L'expression du récepteur au folate par la lignée cellulaire NuTu-19 et les lésions de carcinose a été confirmée par Azaïs et al.(Int J Gynecol Cancer 2015).

Les mesures de fluorescence *in vivo* ont été réalisées 4 heures après l'administration du photosensibilisateur à la dose de 4 mg/Kg par voie intrapéritonéale. Le protocole de détection a été mis en œuvre immédiatement après une anesthésie à l'isoflurane par voie coelioscopique pour visualiser la fluorescence émise par les lésions de carcinose (photodiagnostic).

**III. Photodiagnostic**

Le composé Pyro-PEG-FA de formule (I) a été détecté en fluorescence avec le dispositif médical de cœlioscopie Olympus® (EVIS EXERA II), présentant une fonction PDD (photodiagnostic). L'optique de cœlioscopie utilisée était un cystoscope Olympus® de 4 mm présentant un angle de vue de 30°.

La fonction PDD a été élaborée par Olympus® pour le diagnostic de tumeurs de vessie non visibles en lumière blanche (carcinomes *in situ,* dysplasies, petites tumeurs focales). L'administration d'un précurseur de la protoporphyrine IX (PpIX), l'acide 5-aminolévulinique (5-ALA) et plus récemment l'hexyl-aminolévulinate (HAL, Hexvix®), a permis de visualiser de façon spécifique les lésions vésicales qui apparaissent fluorescentes (rouge) en lumière bleue.

La source lumineuse était une source Xénon. Un premier système de filtre a permis une excitation lumineuse bleue entre 380 et 440 nm. Un second filtre jaune a permis d'accentuer le contraste entre la lumière bleue et la fluorescence rouge émise entre 625 et 655 nm, sachant que ce filtre permettait l'observation optimale d'une fluorescence émise autour de 640 nm. La fluorescence rouge émise par la PpIX étant trop faible par rapport à la lumière bleue, ce filtre jaune a été mis en place au niveau de la caméra afin d'accentuer le contraste entre le bleu et le rouge et de permettre une bonne visualisation des lésions.

Quatre heures après l'injection intra-péritonéale, une anesthésie générale a été réalisée par inhalation continue d'Isoflurane (concentration variant de 1,5 à 3%). Une open cœlioscopie a été réalisée sur la ligne médiane permettant

l'introduction d'un trocart de 5 mm. Une bourse a été réalisée au Vicryl© 2.0 afin de maintenir l'étanchéité autour du trocart. L'insufflation a été mise en place sur le robinet de ce trocart avec un débit de 0,2 L/min de $CO_2$ permettant d'obtenir un pneumopéritoine constant de 3 mmHg. L'introduction d'un cœlioscope de 5 mm à travers ce trocart a permis d'explorer la cavité péritonéale, d'abord en lumière blanche puis en lumière bleue.

La procédure a été réalisée sur 3 rats après administration du PS par voie intrapéritonéale à la dose de 4 mg/Kg.

La figure 4A représente l'image de cœlioscopie obtenue chez un rat immunocompétent ayant développé une carcinose péritonéale après injection IP de cellules NuTu-19 (lignée cellulaire d'adénocarcinome ovarien syngénique de rat). La lésion de carcinose apparaît en blanc. La figure 4B correspond à la figure 4A en lumière blanche.

**Revendications**

1. Composé de formule (I) :

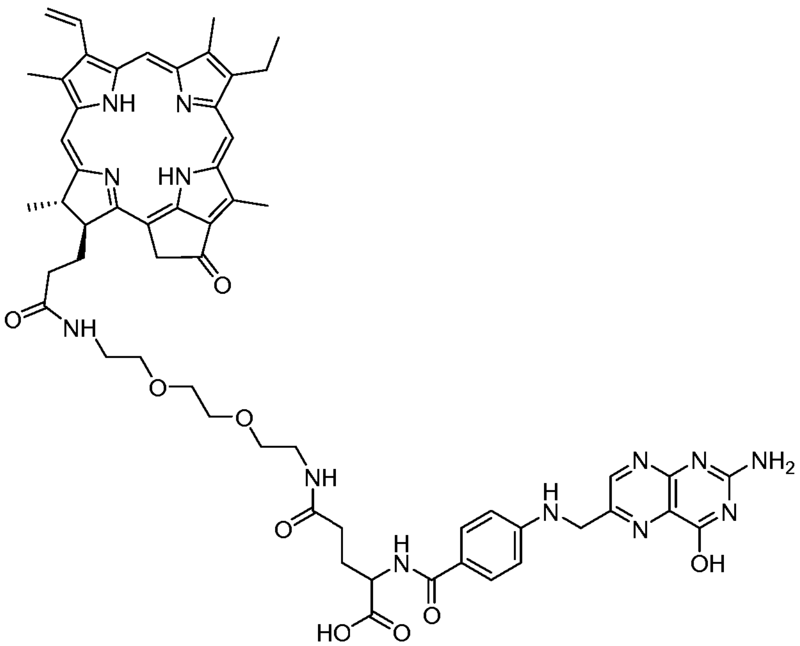

(I),

et ses sels pharmaceutiquement acceptables.

2. Composé selon la revendication 1, pour son utilisation en tant que médicament.

3. Composé selon la revendication 1, pour son utilisation dans le traitement du cancer.

4. Composé pour son utilisation selon la revendication 3, dans le traitement du cancer par thérapie photodynamique.

5. Composé pour son utilisation selon les revendications 3 ou 4, dans la réduction du risque de développer des métastases.

6. Composé pour son utilisation selon la revendication 3 ou 4, dans laquelle le cancer est choisi parmi un cancer des ovaires, des poumons, du rein, de l'endomètre, colorectal, du sein, cancer du pancréas, du cerveau, gastrique, du foie, de la prostate, des testicules, de la vessie, ou de la tête et du cou.

7. Composé pour son utilisation selon la revendication 6, dans laquelle le cancer est un cancer des ovaires.

8. Composé pour son utilisation selon l'une quelconque des revendications 2 à 7, dans laquelle ledit composé est destiné à être administré par voie intrapéritonéale ou par voie intraveineuse.

9. Procédé de préparation d'un composé de formule (I) :

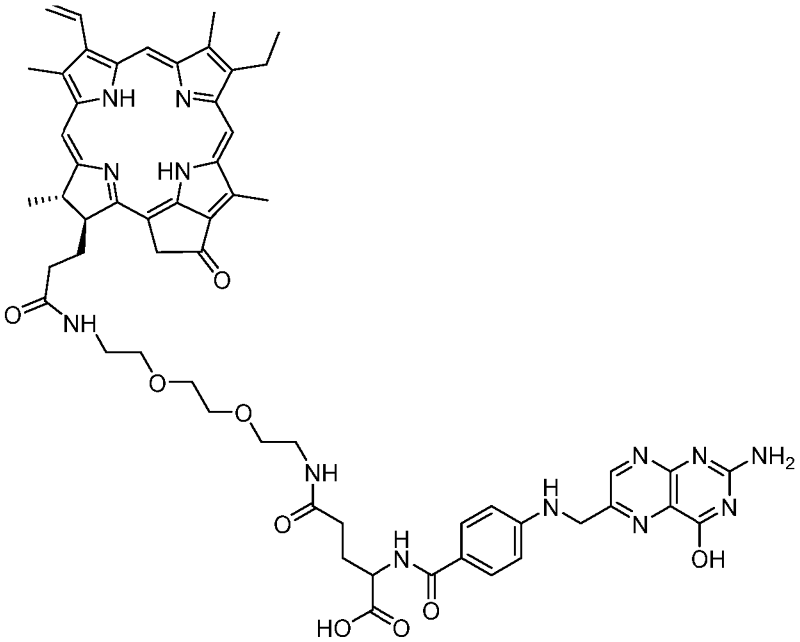

(I),

**caractérisé en ce que** le procédé comprend une étape de couplage entre un composé de formule (II) :

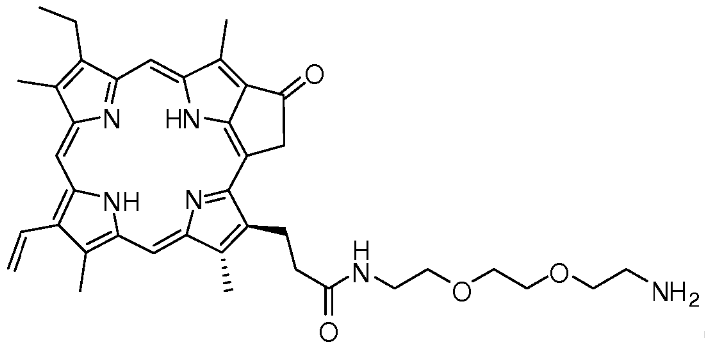

(II),

et l'acide folique.

10. Procédé de préparation d'un composé de formule (I) selon la revendication 9, **caractérisé en ce qu'**il comprend les étapes suivantes :

(a) une étape de couplage entre un composé de formule (IV) :

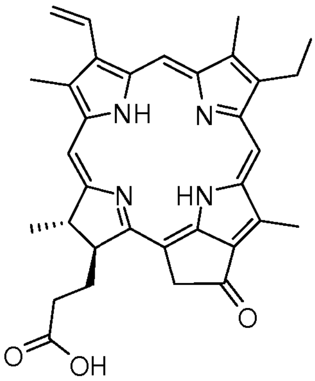

(IV),

avec du N-Boc-2,2'-(éthylènedioxy)diéthylamine, pour obtenir un composé de formule (III) :

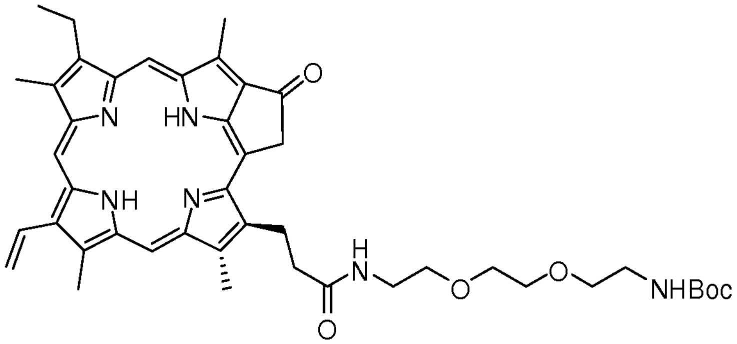

(III),

b) une étape de déprotection du composé de formule (III) obtenu à l'étape a) pour obtenir un composé de formule (II) :

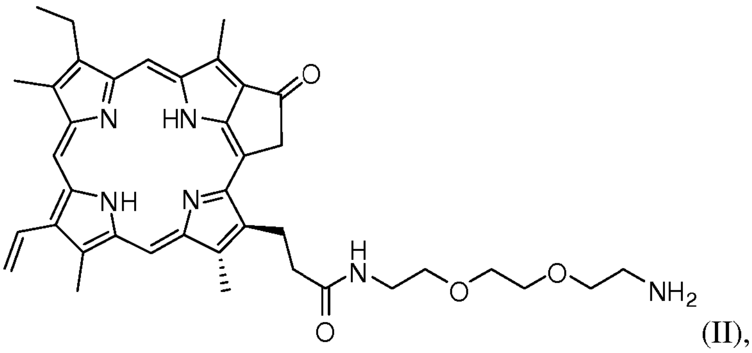

(II),

et

c) une étape de couplage entre le composé de formule (II) obtenu à l'étape b) et l'acide folique pour obtenir un composé de formule (I) :

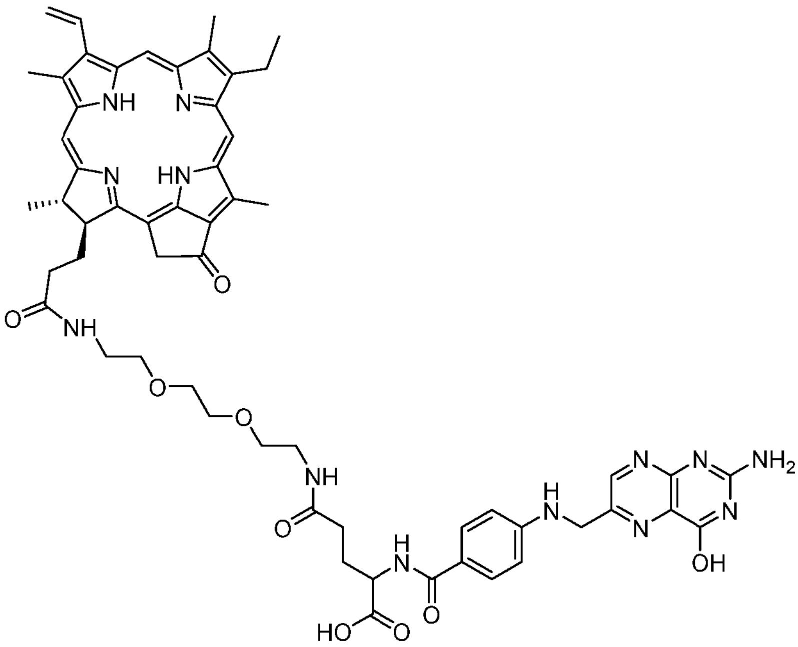

(I).

**11.** Utilisation du composé de formule (I) selon la revendication 1 en tant que marqueur fluorescent.

**12.** Méthode d'imagerie chez un sujet comprenant la visualisation de la fluorescence émise par un composé de formule (I) selon la revendication 1, ledit composé de formule (I) étant préalablement administré audit sujet et photoactivé par une source lumineuse.

## Patentansprüche

**1.** Verbindung der Formel (I):

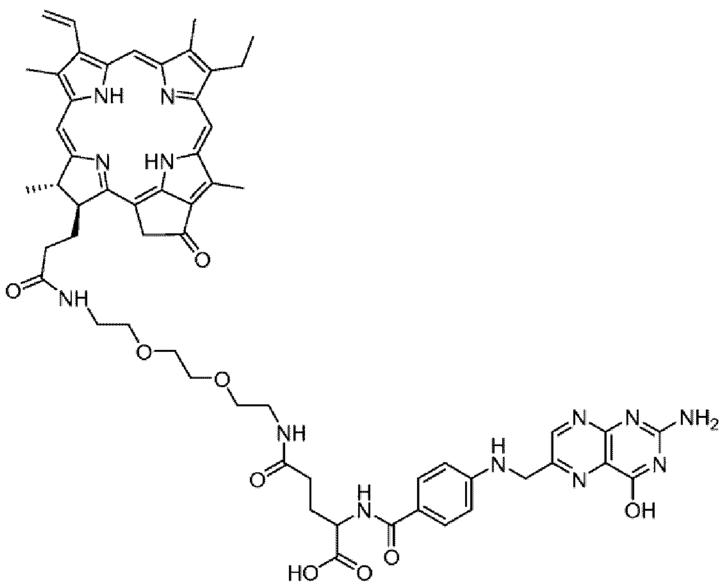

(I),

und deren pharmazeutisch akzeptable Salze.

**2.** Verbindung nach Anspruch 1, zur Verwendung als Medikament.

**3.** Verbindung nach Anspruch 1 zur Verwendung bei der Behandlung von Krebs.

4. Verbindung zur Verwendung nach Anspruch 3, bei der Behandlung von Krebs durch photodynamische Therapie.

5. Verbindung zur Verwendung nach Anspruch 3 oder 4 zur Verringerung des Risikos der Entwicklung von Metastasen.

6. Verbindung zur Verwendung nach Anspruch 3 oder 4, wobei der Krebs ausgewählt ist aus Eierstock-, Lungen-, Nieren-, Endometrium-, Kolorektal-, Brust-, Pankreas-, Gehirn-, Magen-, Leber-, Prostata-, Hoden-, Blasenkrebs oder Krebs im Kopf- oder Halsbereich.

7. Verbindung zur Verwendung nach Anspruch 6, wobei der Krebs Eierstockkrebs ist.

8. Verbindung zur Verwendung nach einem der Ansprüche 2 bis 7, wobei die Verbindung zur intraperitonealen oder intravenösen Verabreichung bestimmt ist.

9. Verfahren zur Herstellung einer Verbindung der Formel (I):

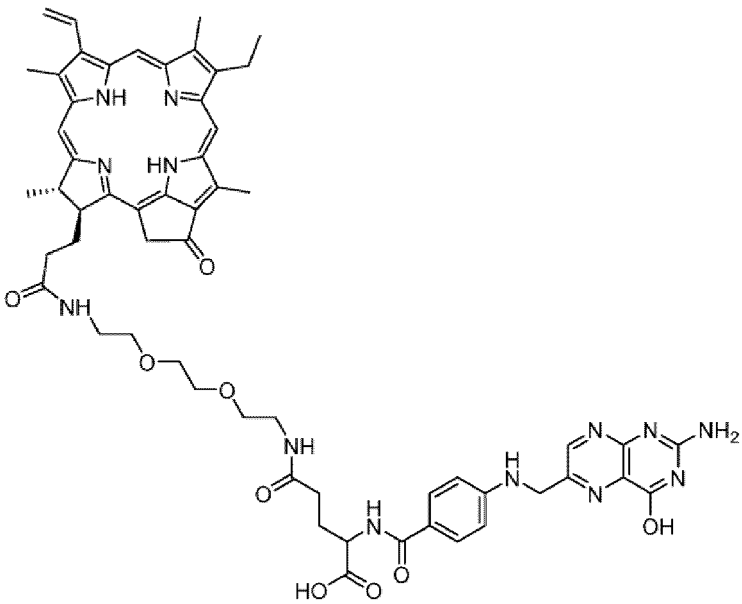

(I),

**dadurch gekennzeichnet, dass** das Verfahren einen Schritt des Koppelns zwischen einer Verbindung der Formel (II):

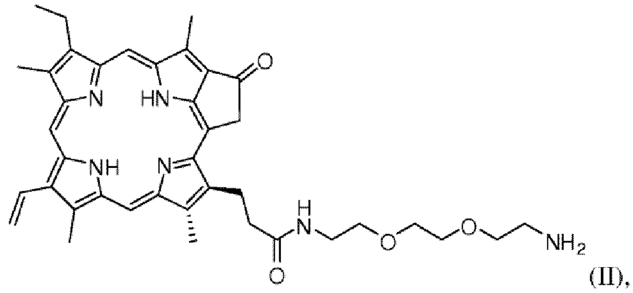

(II),

und Folsäure umfasst.

10. Verfahren zur Herstellung einer Verbindung der Formel (I) nach Anspruch 9, **dadurch gekennzeichnet, dass** es die folgenden Schritte umfasst:

(a) einen Schritt des Koppelns zwischen einer Verbindung der Formel (IV):

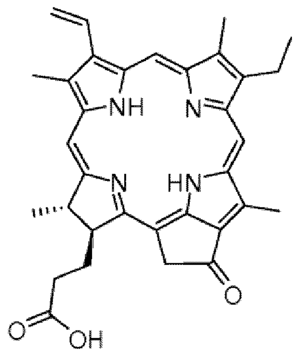

(IV),

und N-Boc-2,2'-(Ethylendioxy)diethylamin, um eine Verbindung der Formel (III) zu erhalten:

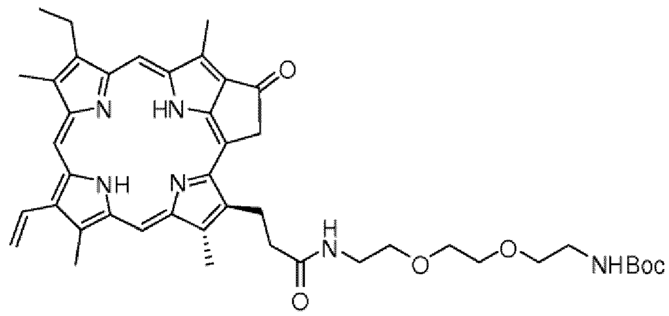

(III),

(b) einen Schritt des Entschützens der in Schritt a) erhaltenen Verbindung der Formel (III), um eine Verbindung der Formel (II) zu erhalten:

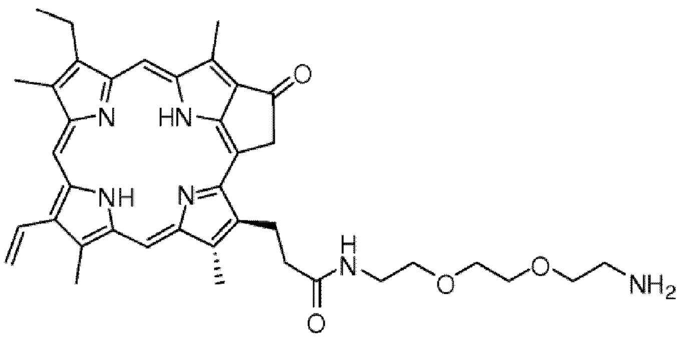

(II),

(c) einen Schritt des Koppelns zwischen der in Schritt b) erhaltenen Verbindung der Formel (II) und Folsäure, um eine Verbindung der Formel (I) zu erhalten:

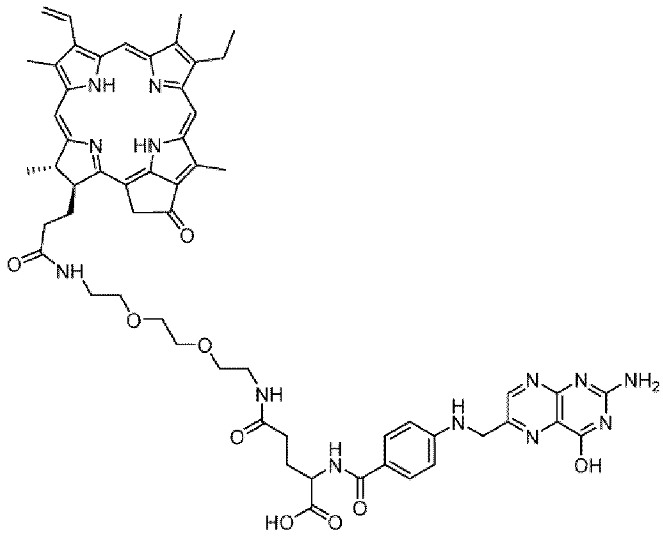

(I).

11. Verwendung der Verbindung der Formel (I) nach Anspruch 1 als Fluoreszenzmarker.

12. Verfahren zur Bildgebung in einem Individuum, umfassend das Sichtbarmachen der von einer Verbindung der Formel (I) nach Anspruch 1 emittierten Fluoreszenz, wobei die Verbindung der Formel (I) dem Individuum zuvor verabreicht und durch eine Lichtquelle photoaktiviert wurde.

**Claims**

1. A compound of formula (I):

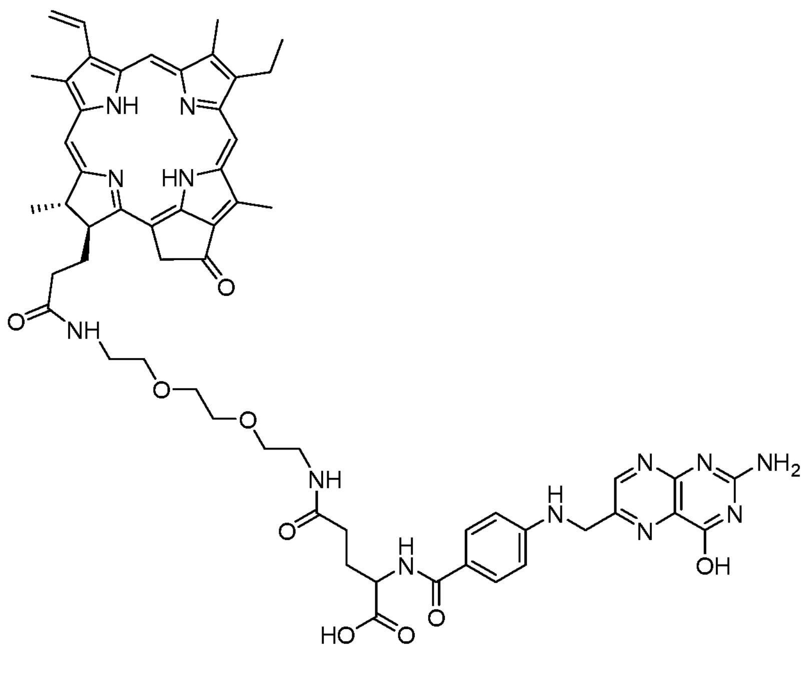

(I),

and the pharmaceutically acceptable salts thereof.

2. The compound as claimed in claim 1, for use thereof as a medicament.

3. The compound as claimed in claim 1, for use thereof in the treatment of cancer.

**4.** The compound for use thereof as claimed in claim 3, in the treatment of cancer by photodynamic therapy.

**5.** The compound for use thereof as claimed in claim 3 or 4, in the reduction of the risk of developing metastases.

**6.** The compound for use thereof as claimed in claim 3 or 4, wherein the cancer is chosen from ovarian, lung, kidney, endometrial, colorectal or breast cancer, pancreatic cancer, brain, gastric, liver, prostate, testicular, bladder, or head and neck cancer.

**7.** The compound for use thereof as claimed in claim 6, wherein the cancer is ovarian cancer.

**8.** The compound for use thereof as claimed in any one of claims 2 to 7, wherein said compound is intended to be administered intraperitoneally or intravenously.

**9.** A process for preparing a compound of formula (I):

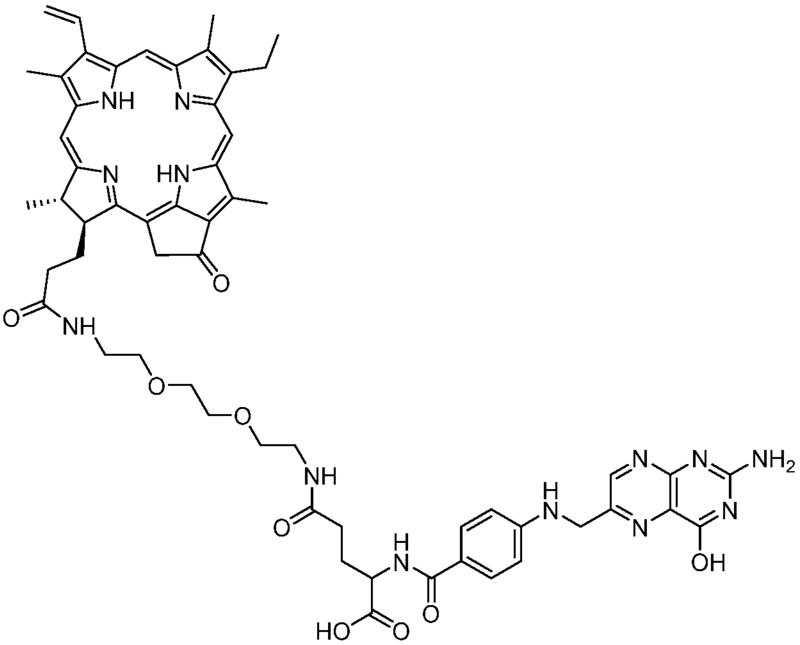

(I),

**characterized in that** the process comprises a step of coupling between a compound of formula (II):

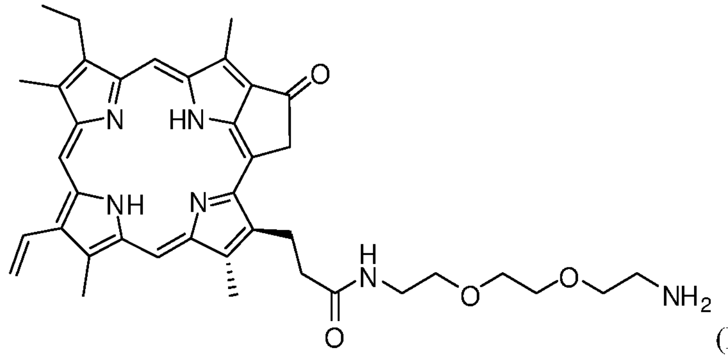

(II),

and folic acid.

**10.** The process for preparing a compound of formula (I) as claimed in claim 9, **characterized in that** it comprises the following steps:

(a) a step of coupling between a compound of formula (IV):

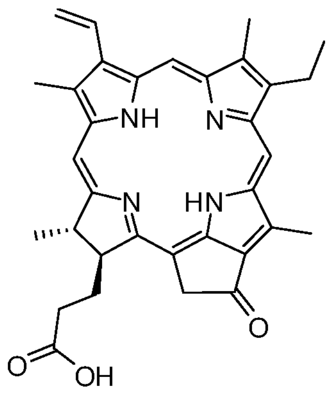

(IV),

and N-Boc-2,2'-(ethylenedioxy)diethylamine, so as to obtain a compound of formula (III):

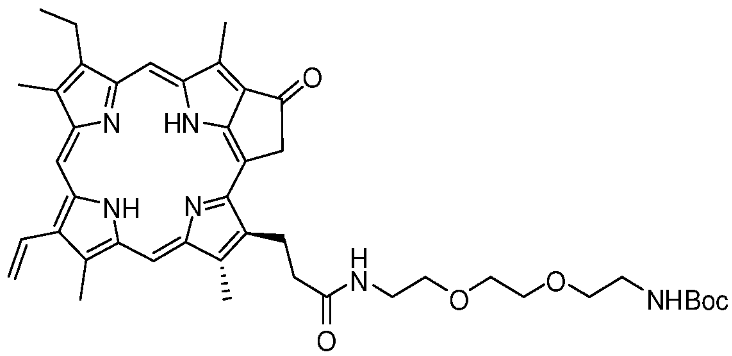

(III),

b) a step of deprotection of the compound of formula (III) obtained in step a) so as to obtain a compound of formula (II):

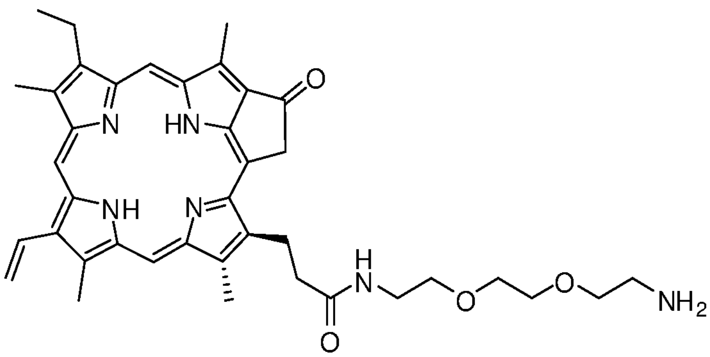

(II),

and

c) a step of coupling between the compound of formula (II) obtained in step b) and folic acid so as to obtain a compound of formula (I):

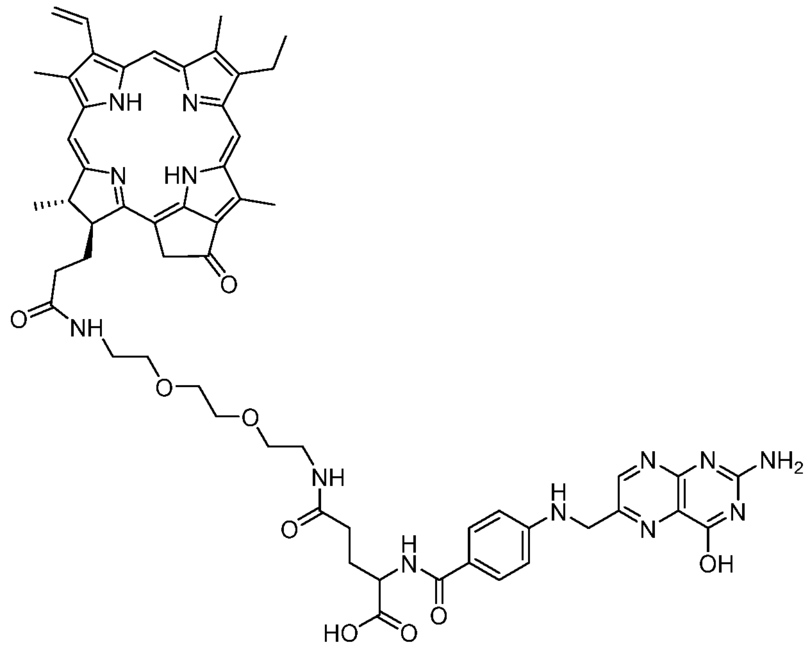

(I).

**11.** The use of the compound of formula (I) as claimed in claim 1 as a fluorescent marker.

**12.** A method for imaging in a subject, comprising the visualization of the fluorescence emitted by a compound of formula (I) as claimed in claim 1, said compound of formula (I) being previously administered to said subject and photoactivated by a light source.

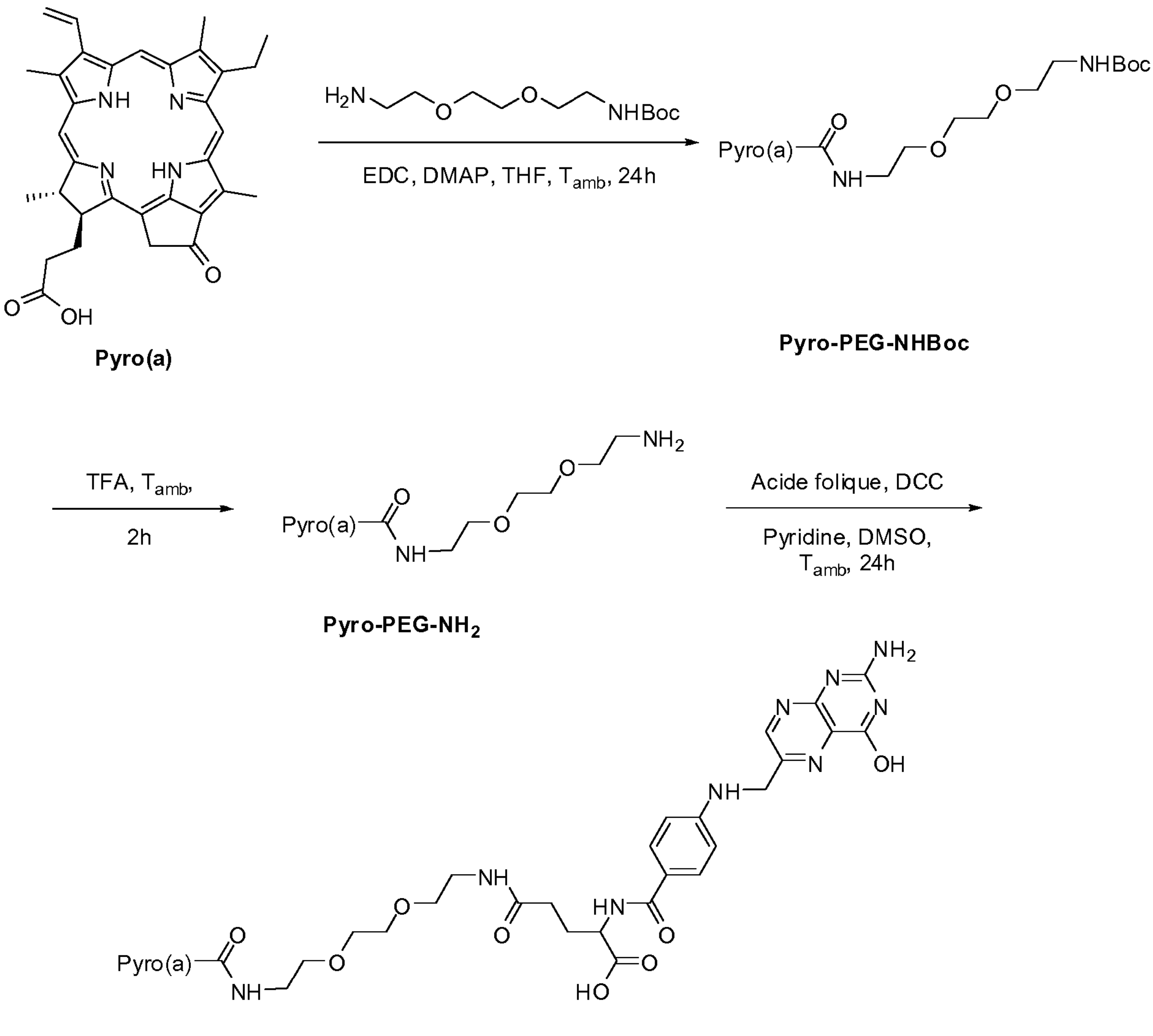


**Pyro(a)**

**Pyro-PEG-NHBoc**

**Pyro-PEG-$NH_2$**

**Pyro-PEG-FA**

**FIGURE 1**

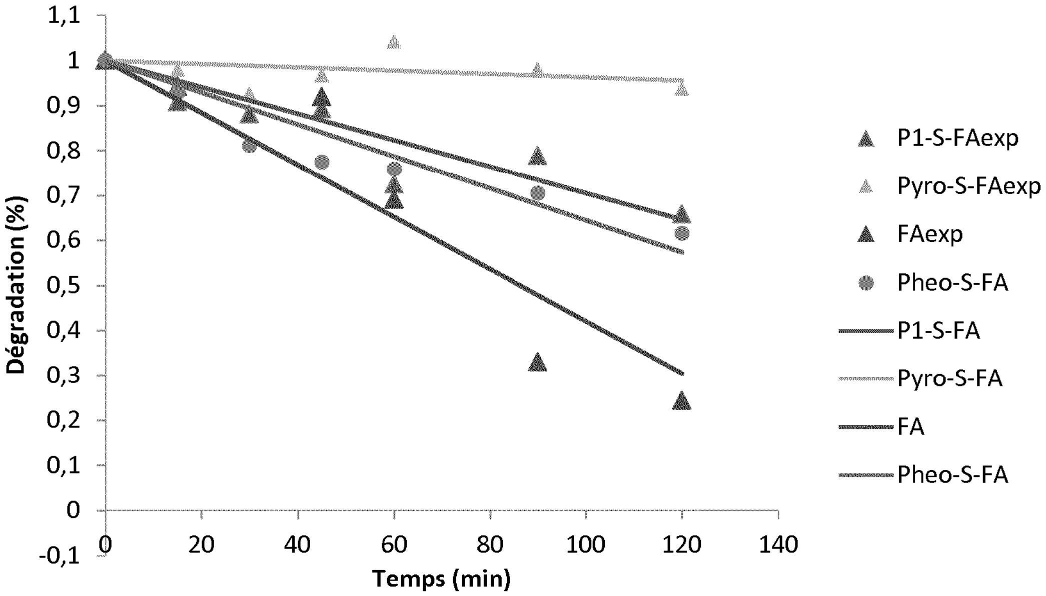
1,1
1
0,9
0,8
0,7
0,6
0,5
0,4
0,3
0,2
0,1
0
-0,1
Dégradation (%)
0
20
40
60
80
100
120
140
Temps (min)
P1-S-FAexp
Pyro-S-FAexp
FAexp
Pheo-S-FA
P1-S-FA
Pyro-S-FA
FA
Pheo-S-FA

**FIGURE 2**

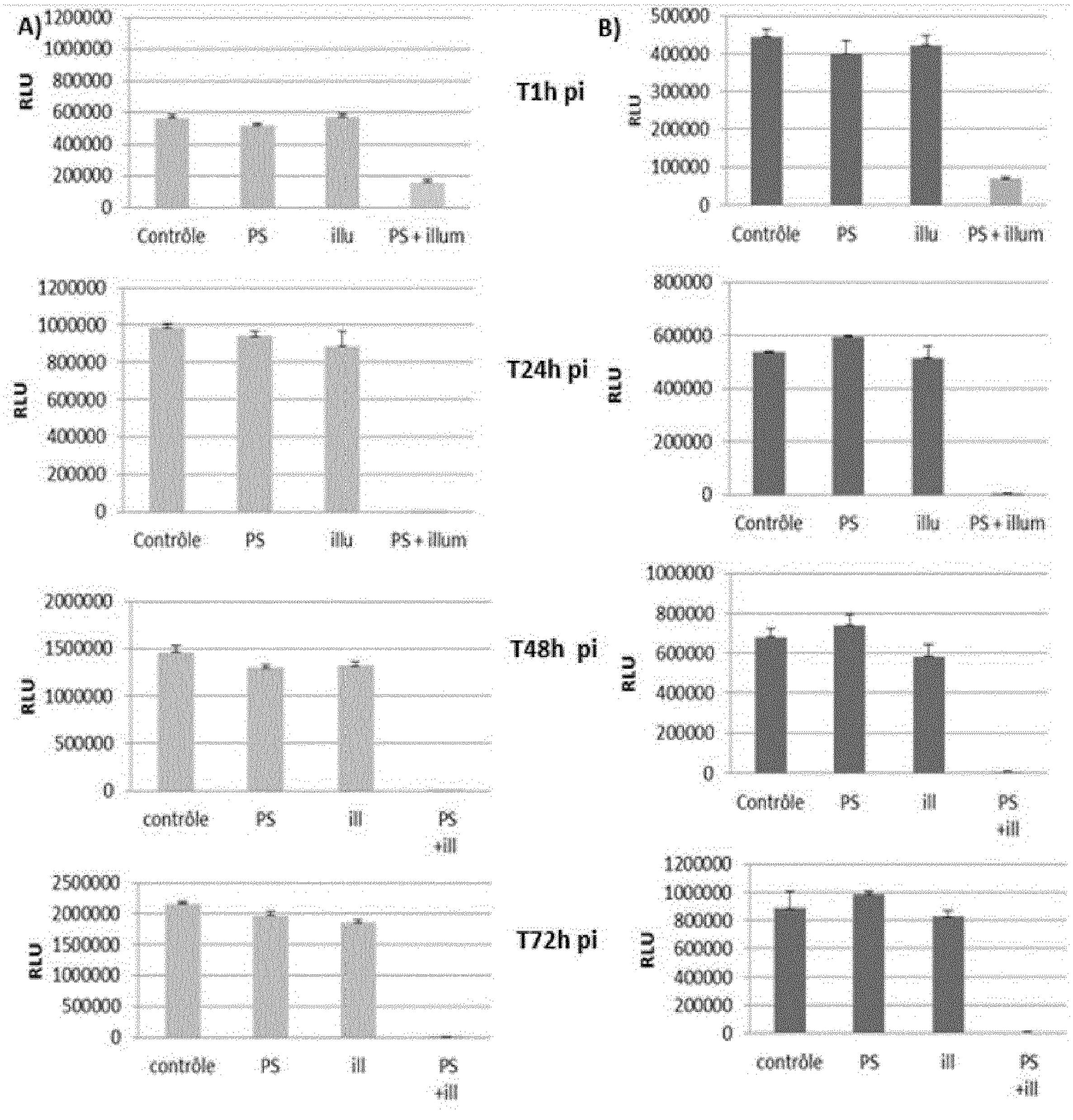
A)
B)
T1h pi
T24h pi
T48h pi
T72h pi
RLU
Contrôle
PS
illu
PS + illum
ill
PS +ill

FIGURE 3A

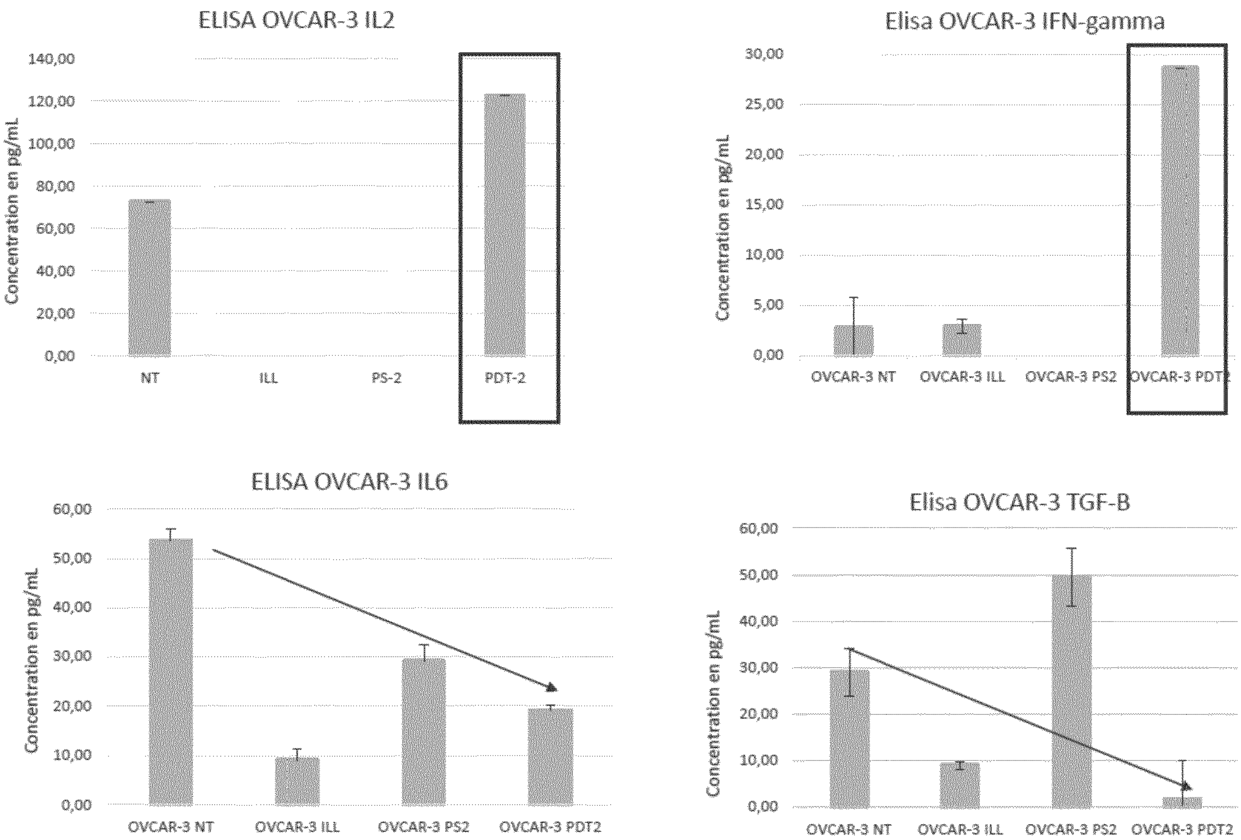
ELISA OVCAR-3 IL2
Concentration en pg/mL
140,00
120,00
100,00
80,00
60,00
40,00
20,00
0,00
NT
ILL
PS-2
PDT-2
Elisa OVCAR-3 IFN-gamma
Concentration en pg/mL
30,00
25,00
20,00
15,00
10,00
5,00
0,00
OVCAR-3 NT
OVCAR-3 ILL
OVCAR-3 PS2
OVCAR-3 PDT2
ELISA OVCAR-3 IL6
Concentration en pg/mL
60,00
50,00
40,00
30,00
20,00
10,00
0,00
OVCAR-3 NT
OVCAR-3 ILL
OVCAR-3 PS2
OVCAR-3 PDT2
Elisa OVCAR-3 TGF-B
Concentration en pg/mL
60,00
50,00
40,00
30,00
20,00
10,00
0,00
OVCAR-3 NT
OVCAR-3 ILL
OVCAR-3 PS2
OVCAR-3 PDT2

**FIGURE 3B**

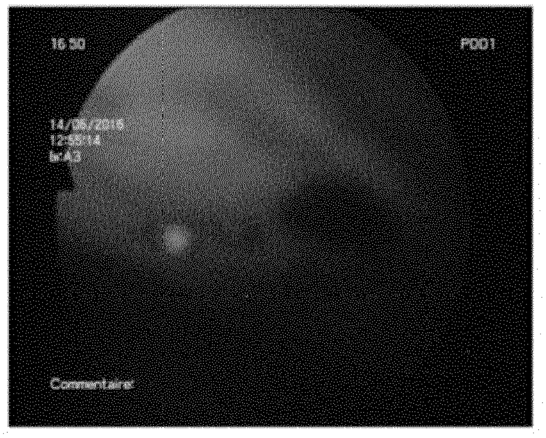


FIGURE 4A

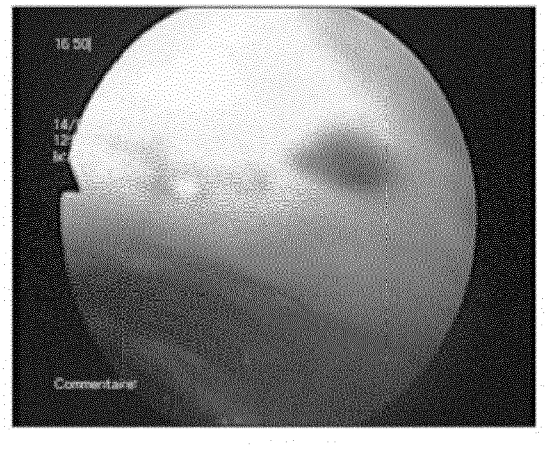


FIGURE 4B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **SCHNEIDER et al.** *Bioorganic & Médicinal Chemistry,* 2005, vol. 13, 2799-2808
- **AZAÏS et al.** *Photodiagnosis and Photodynamic Therapy,* 2016, vol. 13, 130-138
- **YOU et al.** *Bioorganic & Médicinal Chemistry,* 2015, vol. 23, 1453-1462
- **BERGE et al.** *J. Pharm. Sci.,* 1977, vol. 66 (1), 1-19
- **MUNCK et al.** *Photodiagnosis and Photodynamic Therapy,* 2016, vol. 16, 23-26
- **GUYON et al.** *Journal of Biomédical Optics,* 2012, vol. 17 (3
- **ASSARAF et al.** *Drug résistance Updates,* 2014, vol. 17, 89-95
- **ROSE et al.** *Am. J. Obstet. Gynecol.,* 1996, vol. 175 (3), 593-9
- **AZAÏS et al.** *Int J Gynecol Cancer,* 2015